# EUROPEAN PATENT SPECIFICATION

(11) **EP 4 106 680 B1**
(45) Date of publication and mention of the grant of the patent: **03.07.2024**
(21) Application number: 21776497.6
(22) Date of filing: 23.03.2021
(51) Int. Cl.: A61F 2/44

(54) **MOTION PRESERVING SPINAL IMPLANT FOR TOTAL DISC REPLACEMENT**
BEWEGUNGSERHALTENDES WIRBELSÄULENIMPLANTAT FÜR BANDSCHEIBENTOTALERSATZ
IMPLANT RACHIDIEN PRÉSERVANT LE MOUVEMENT POUR REMPLACEMENT TOTAL DE DISQUE

(30) Priority: 23.03.2020 US 202016826742; 27.07.2020 US 202016940234
(43) Date of publication of application: 28.12.2022
(73) Proprietor: Spinvention, LLC, Naples, FL 34120 (US)
(72) Inventor: MAKWANA, Nayan Manharlal, Naples, Florida 34120 (US)
(74) Representative: Loo, Chi Ching
(86) International application number: PCT/US2021/023721
(87) International publication number: WO 2021/195107

(56) References cited:
- EP-A1- 0 642 775
- WO-A2-2010/030756
- FR-A1- 2 851 157
- US-A1- 2002 035 400
- US-A1- 2006 293 752
- US-A1- 2007 021 836
- US-A1- 2013 158 665
- US-A1- 2019 008 651
- US-B1- 10 758 362
- US-B1- 10 758 362
- US-B2- 7 799 083
- US-B2- 8 172 904
- US-B2- 8 613 768
- US-B2- 9 220 603

## Description

### FIELD OF THE INVENTION

The present invention relates generally to human body implants. More particularly, the present invention relates to a motion preserving spinal implant for replacement of a spinal disc.

### BACKGROUND OF THE INVENTION

Spinal implants are intended to treat degenerative disc disease (DDD) or other disc injuries. Spinal fusion treatment is a widely used treatment to alleviate pain, but limits range of motion and mobility for a patient. Total disc replacement is another treatment for disc degenerative disease that aims to preserve motion and limit complications related to spinal fusion such as adjacent level wear and disc degeneration. Total disc replacement is an effective solution for degenerative disc disease and gaining interest due to increasing prevalence of neck pain, lower back pain, and pain in general. Thus, there is a need for functional improvement. For an example, without limitations, there is a need for total disc replacement spinal implants that reduce wear due to metal to metal sliding and corrosive surfaces, increase cushioning, improve shock absorption, reduce wear debris of metal, and maintain spinal motion range.

US2002/035400A1 relates to a surgical implant, suitable for use as an intervertebral disc endoprosthesis. The invention has two rigid opposing shells, each having an outer surface adapted to engage the surfaces of the bones of a joint such that the shells are immobilized by friction. The shells may be convex. A central body is disposed in a cavity formed by the shells and provides a range of motion similar to that of a healthy joint.

WO2010030756A3 discloses a modular, six degrees of freedom spatial mechanism for spinal disc prosthesis.

US20070021836A1 relates to a spinal disc prosthetic device having up to six degrees of freedom.

FR2851157A1 relates to an intervertebral prosthesis having a core positioned between an upper plate and a lower plate, the plates enabling the movement of the core within.

US20130158665A1 discloses a spinal implant having a first component, a second component and a core component between the first and second components. The first and second components each engage with vertebrae.

US20190008651A1 relates to a closed profile, total disc replacement device with mechanical features designed to sustain, restrain and guide the larger motions required to preserve normal mechanical motion whilst providing a flexion component to guide and restrain the finer motions reached at the extremes of the mechanical motion preservation components.

EP0642775A1 discloses a preformed prosthesis destined to replace a damaged disc, comprising two outer layers rigidly fixed on the adjacent vertebrae and connected via a compression cushion.

US2006293752A1 relates to an intervertebral disc prosthesis having a first plate and a second plate designed for attachment to upper and lower vertebrae respectively, and a prosthesis core between the plates, as well as a spring connected to the first plate and the second plate.

US7799083B2 discloses a motion restoring prosthesis having inner cooperating articulating surfaces and outer bone engaging surfaces for use in a spinal joint.

US9220603B2 relates to a prosthetic intervertebral disc which includes an upper plate, a lower plate, and a congruent stepped features to provide limited articulating motion between the plates.

According to an aspect of the invention, there is provided a motion preserving spinal implant as recited in claim 1. Optional features are set out in dependent claims 2 to 18.

The present invention solves these problems by providing a treatment solution that reduces degeneration due to metal wear because of no sliding between metal plates, increases cushioning with effective inner core design features, and uses special polymeric and elastomeric materials having varying hardness and physical properties such as silicone or liquid silicon rubber that also provides shock absorption, and maintains range of motion due to effective outer core design, its features, and choice of materials. All components in the assembly are designed such that it can effectively resist compression forces, shear-compression forces, and torsion forces.

Additional advantages of the invention will be set forth in part in the description which follows, and in part will be obvious from the description, or may be learned by practice of the invention. Additional advantages of the invention may be realized and attained by means of the instrumentalities and combinations particularly pointed out in the detailed description of the invention section. Further benefits and advantages of the embodiments of the invention will become apparent from consideration of the following detailed description given with reference to the accompanying drawings, which specify and show preferred embodiments of the present invention.

### BRIEF DESCRIPTION OF THE DRAWINGS

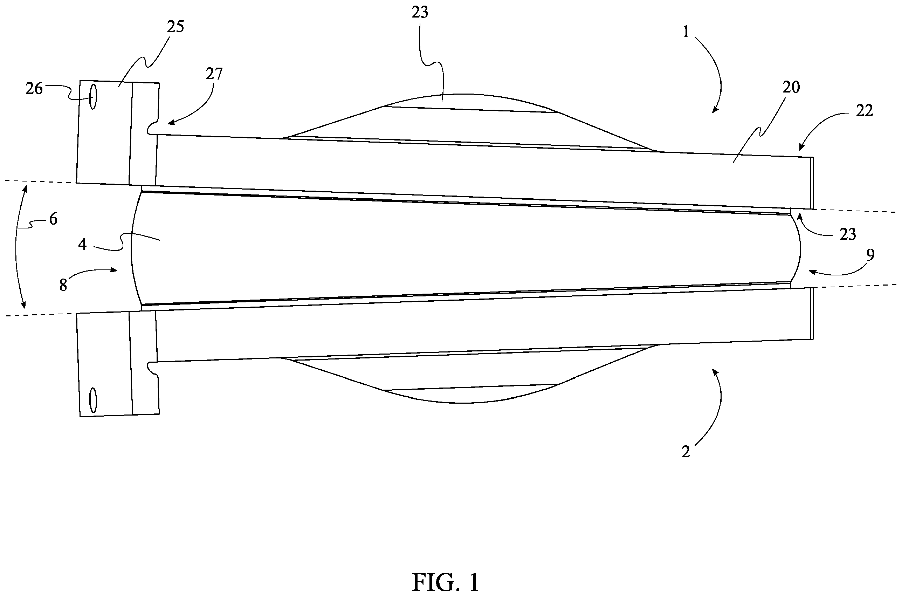

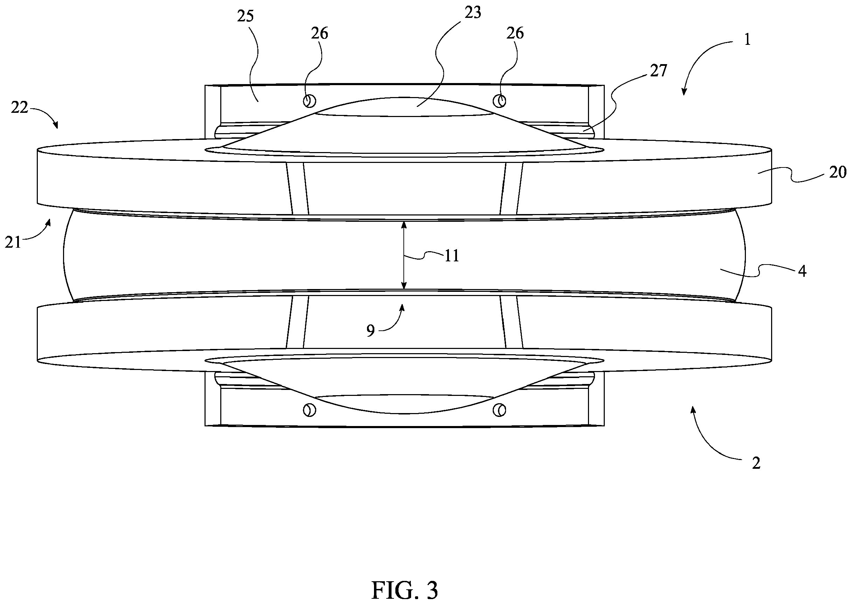

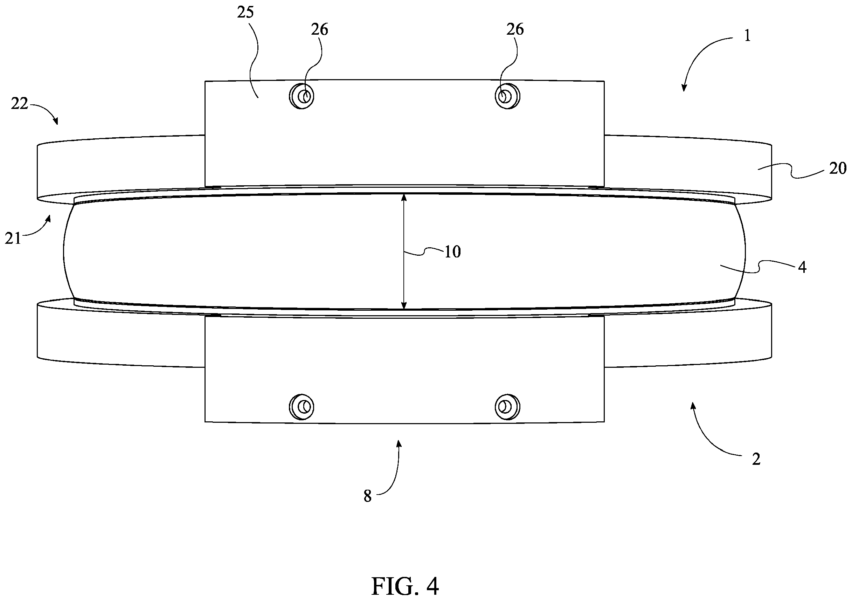

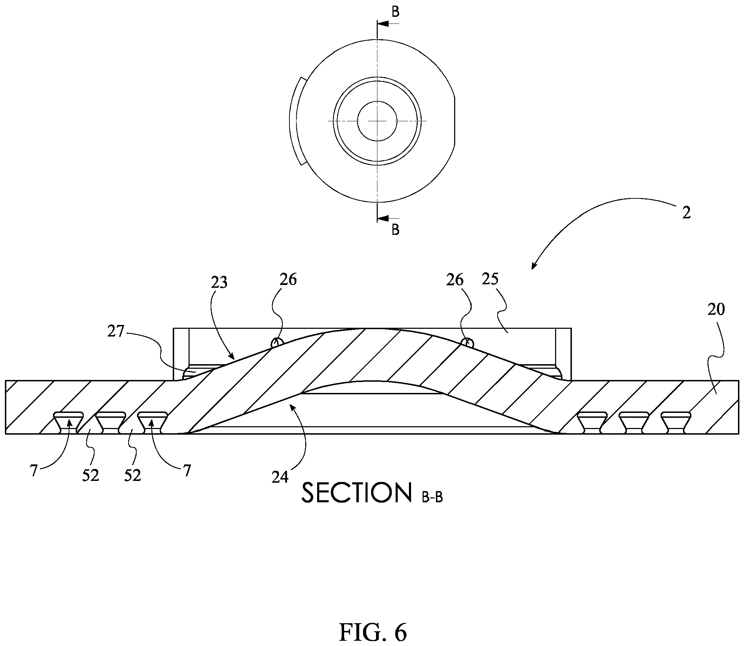

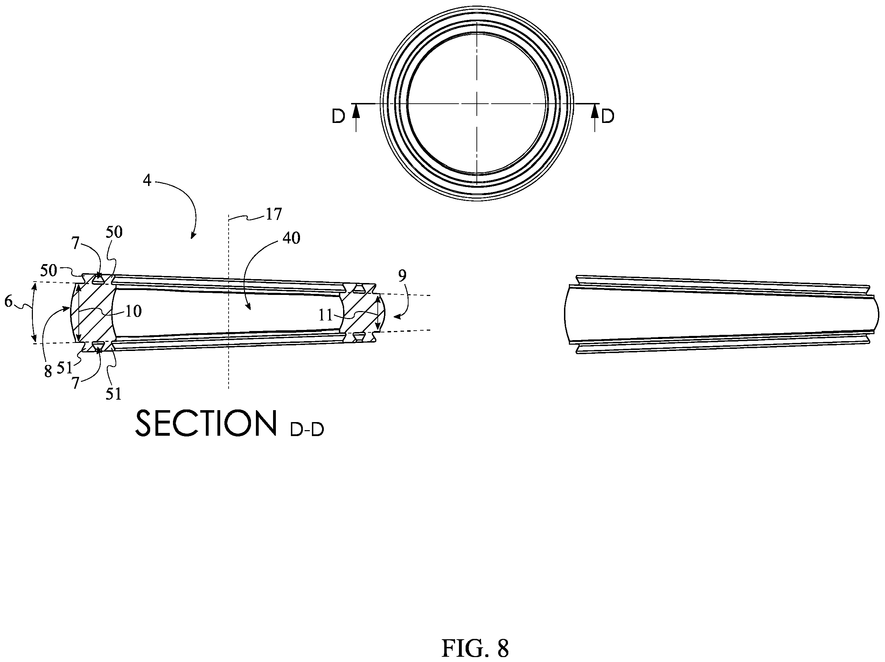

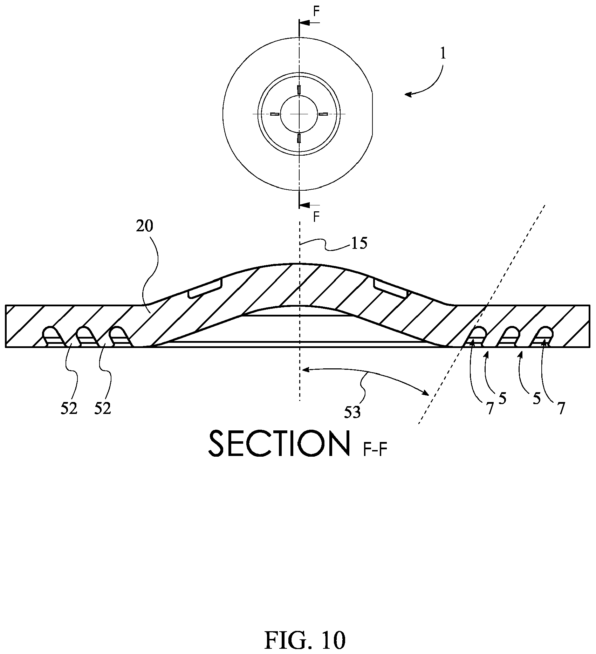

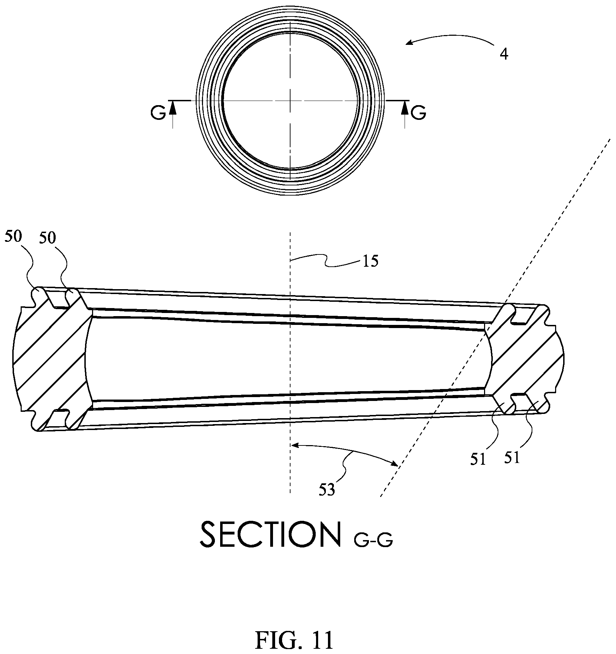

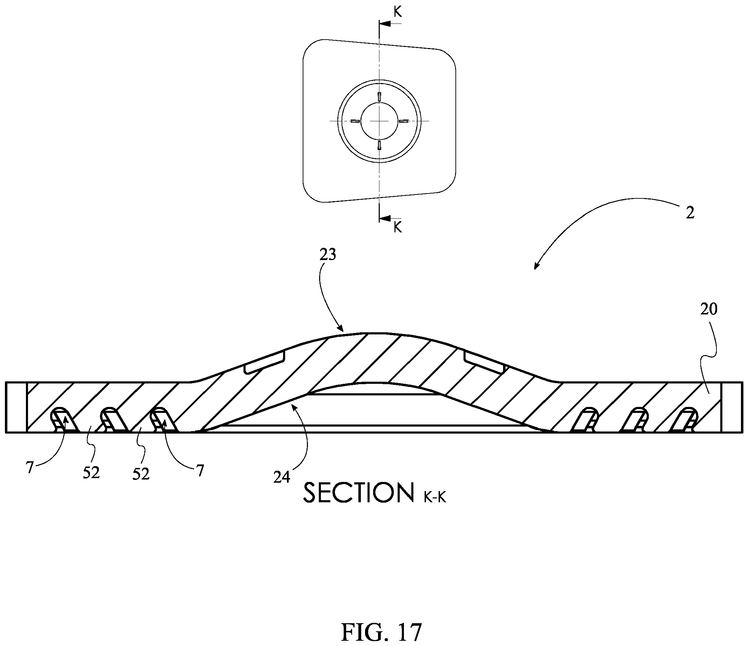

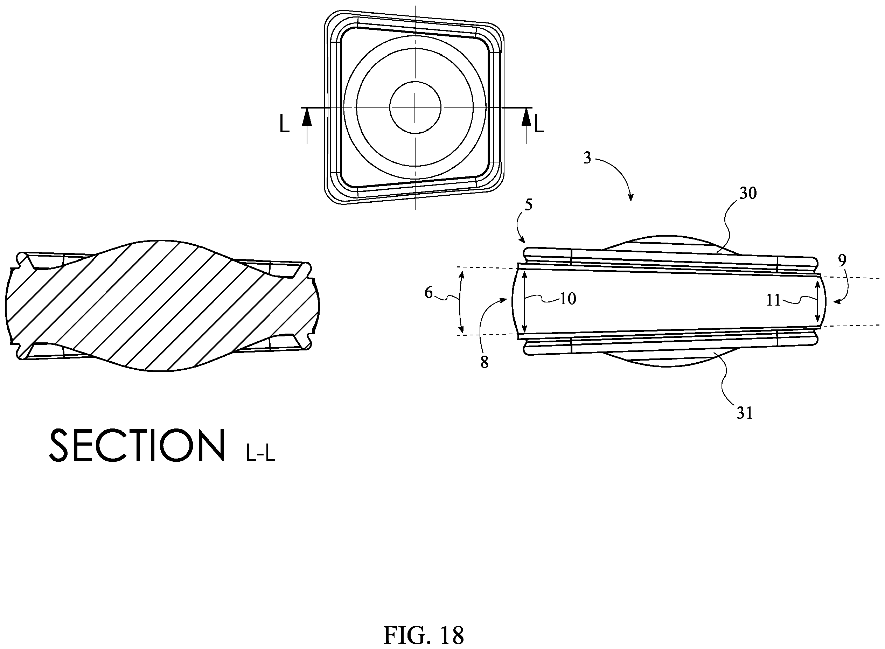

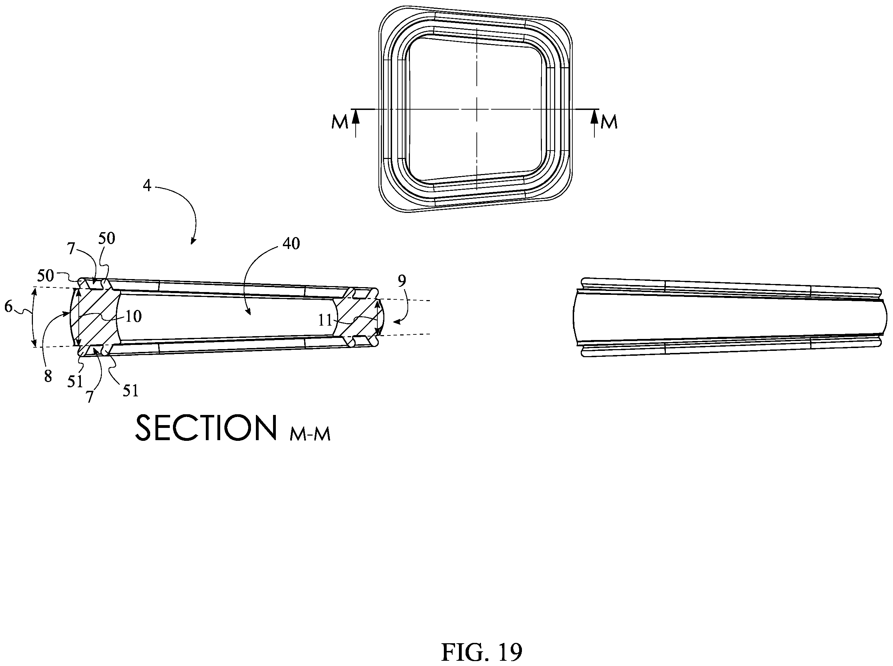

The embodiments of the invention are set forth as "first" and "third alternate embodiments" and are respectively illustrated in the figures 1 to 11 and 16 to 19.

The examples referred to as "second alternate embodiment" and "fourth alternate embodiment" illustrated in the other figures 12 to 15 and 20 to 24, do not form part of the invention but represent background art that is useful for understanding the invention.
FIG. **1** is a side view of the present invention.
FIG. **2** is an exploded perspective view of the present invention.
FIG. **3** is a front view of the present invention.
FIG. **4** is a rear view of the present invention.
FIG. **5** is a top view and a side sectional view of the present invention.
FIG. **6** is a top view and a front sectional view of one of the end plates of the present invention.
FIG. **7** is a top view, a side sectional view, and a side view of the inner core of the present invention.
FIG. **8** is a top view, a side sectional view, and a side view of the outer core of the present invention.
FIG. **9** is a top view and a side sectional view of an alternative embodiment of the present invention incorporating anchoring protrusions.
FIG. **10** is a top view and a side sectional view of one of the end plates in the alternate embodiment.
FIG. **11** is a top view and a side sectional view of the outer core in the alternate embodiment.
FIG. **12** is an exploded perspective view of a second alternate embodiment (not claimed).
FIG. **13** is a top view and a side sectional view of the second alternate embodiment.
FIG. **14** is a top view, a side sectional view, and a side view of the inner core of the second alternate embodiment.
FIG. **15** is a top view, a side sectional view, and a side view of the outer core of the second alternate embodiment.
FIG. **16** is an exploded perspective view of a third alternate embodiment of the present invention.
FIG. **17** is a top view and a side sectional view of one of the end plates of the third alternate embodiment.
FIG. **18** is a top view, a side sectional view, and a side view of the inner core of the third alternate embodiment.
FIG. **19** is a top view, a side sectional view, and a side view of the outer core of the third alternate embodiment.
FIG. **20** is an exploded perspective view of a fourth alternate embodiment (not claimed).
FIG. **21** is a top view and a side sectional view of the fourth alternate embodiment.
FIG. **22** is a top view and a side sectional view of one of the end plates of the fourth alternate embodiment.
FIG. **23** is a top view, a side sectional view, and a side view of the inner core of the fourth alternate embodiment.
FIG. **24** is a top view, a side sectional view, and a side view of the outer core of the fourth alternate embodiment.

### DETAIL DESCRIPTIONS OF THE INVENTION

All illustrations of the drawings are for the purpose of describing selected versions of the present invention and are not intended to limit the scope of the present invention. The present invention is to be described in detail and is provided in a manner that establishes a thorough understanding of the present invention. There may be aspects of the present invention that may be practiced or utilized without the implementation of some features as they are described. It should be understood that some details have not been described in detail in order to not unnecessarily obscure focus of the invention. References herein to "the preferred embodiment", "one embodiment", "some embodiments", or "alternative embodiments" should be considered to be illustrating aspects of the present invention that may potentially vary in some instances, and should not be considered to be limiting to the scope of the present invention as a whole.

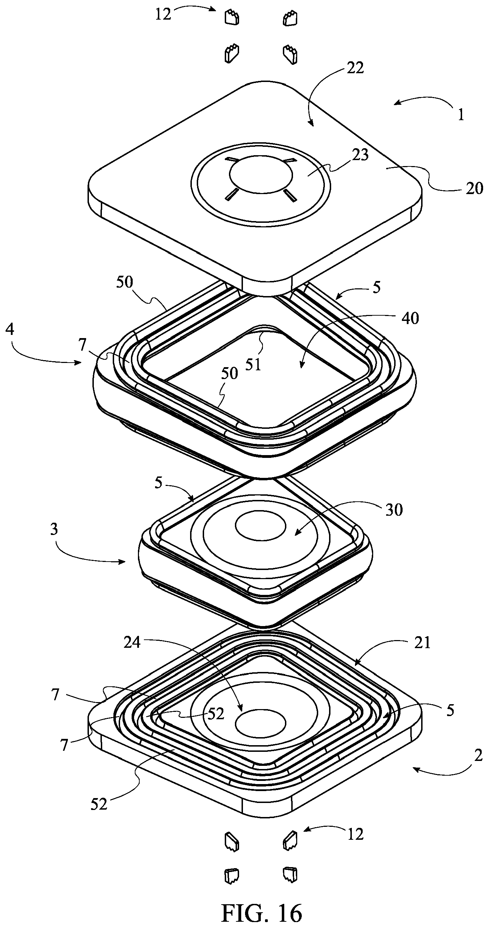

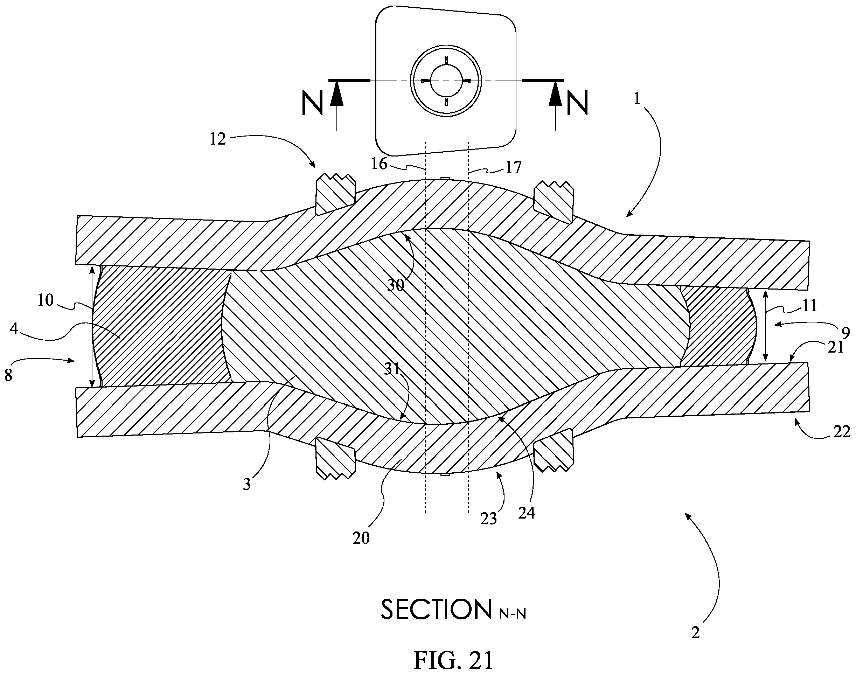

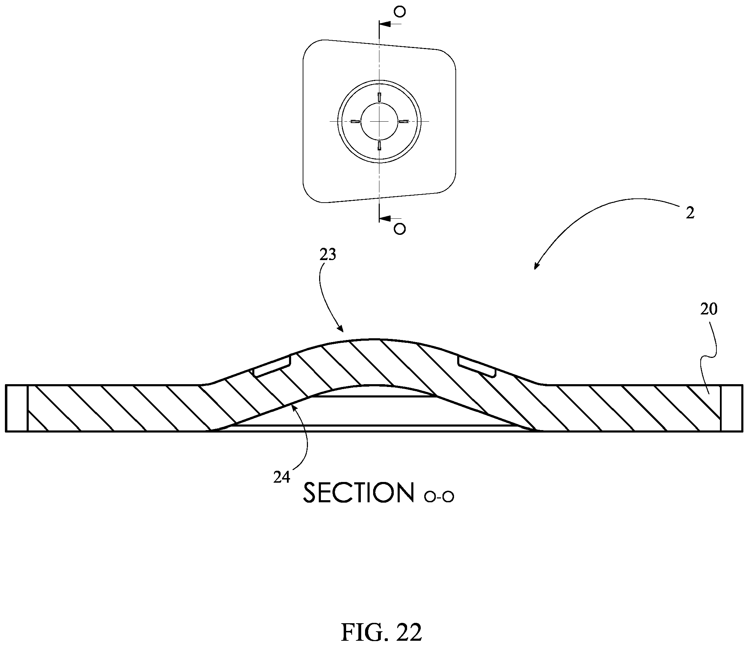

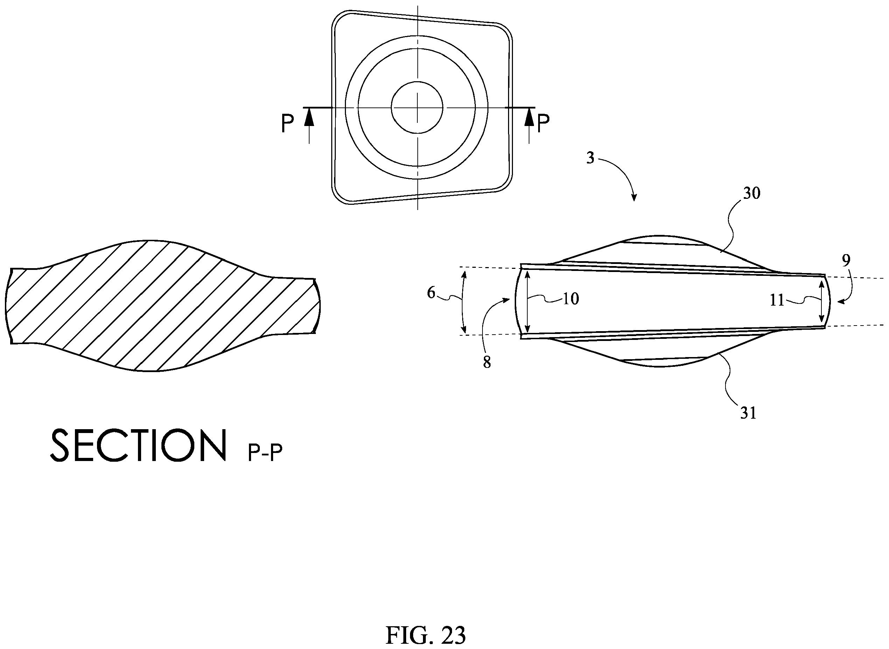

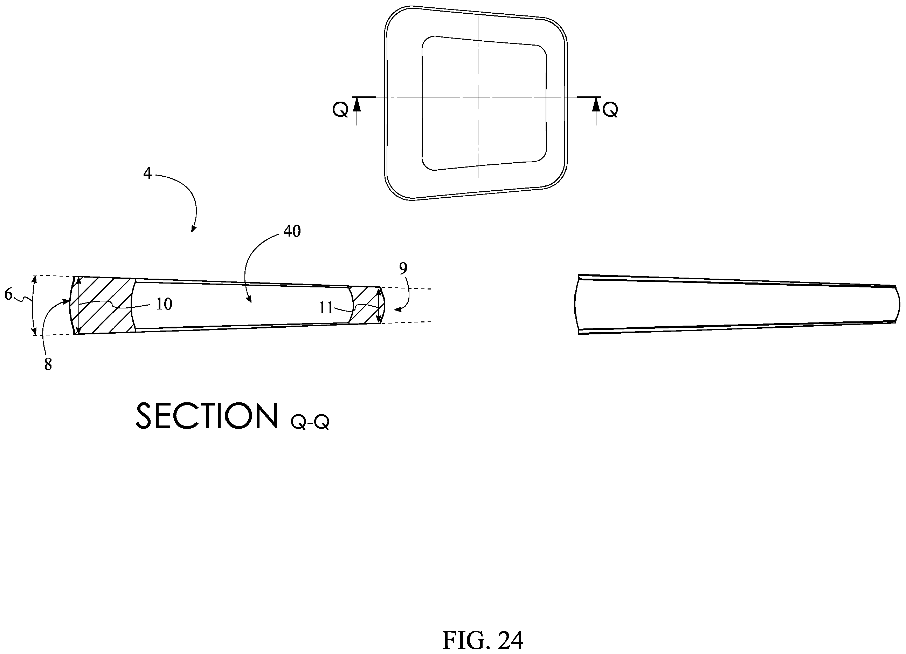

The present invention is a spinal implant intended for use in total replacement of a degenerated spinal disc. In general, referring to FIGS. **1-5****,** the present invention comprises a first end plate **1,** a second end plate **2,** an inner core **3,** and an outer core **4.** In some embodiments of the present invention, the first end plate **1,** the second end plate **2,** the inner core **3,** and the outer core **4** each have generally radial geometry, though it may be noted that other geometries may be utilized as desired or useful. The inner core **3** and the outer core **4** are substantially wider than tall, having a certain thickness, or axial height. As previously mentioned, in some embodiments, the inner core **3** and the outer core **4** each have radial geometry and are generally disc shaped, having a radial axial cross section. In some embodiments, the first end plate **1,** the second end plate **2,** the inner core **3,** and the outer core **4** may have other general geometries. For example, as shown in FIGS. **16-24****,** in some embodiments, the first end plate **1,** the second end plate **2,** the inner core **3,** and the outer core **4** each have generally rectilinear peripheral geometry with rounded corners.

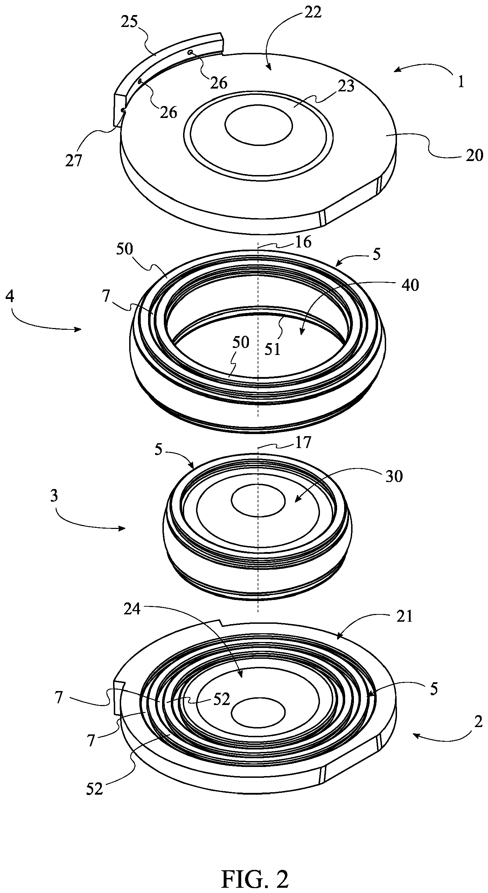

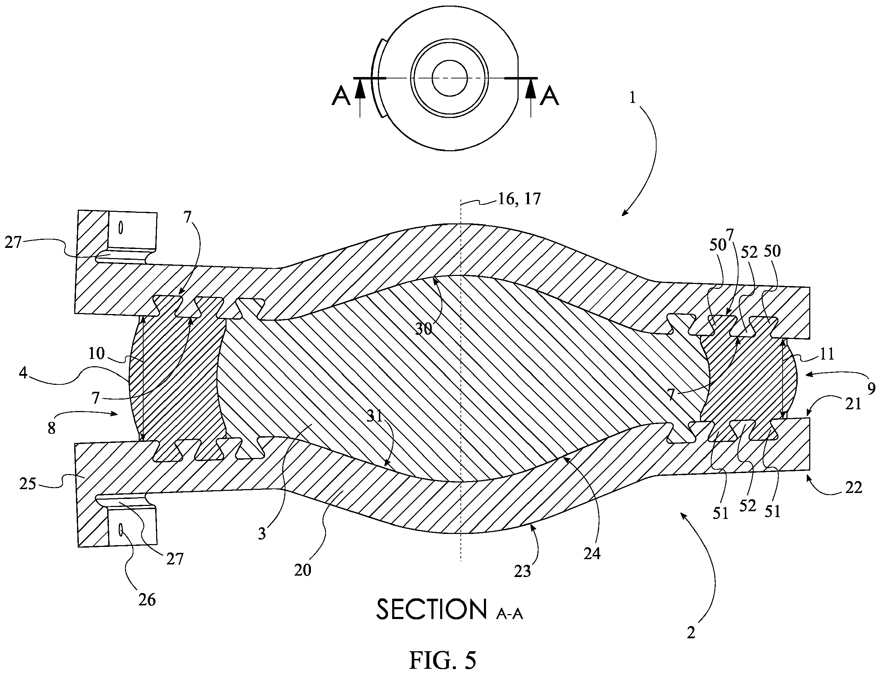

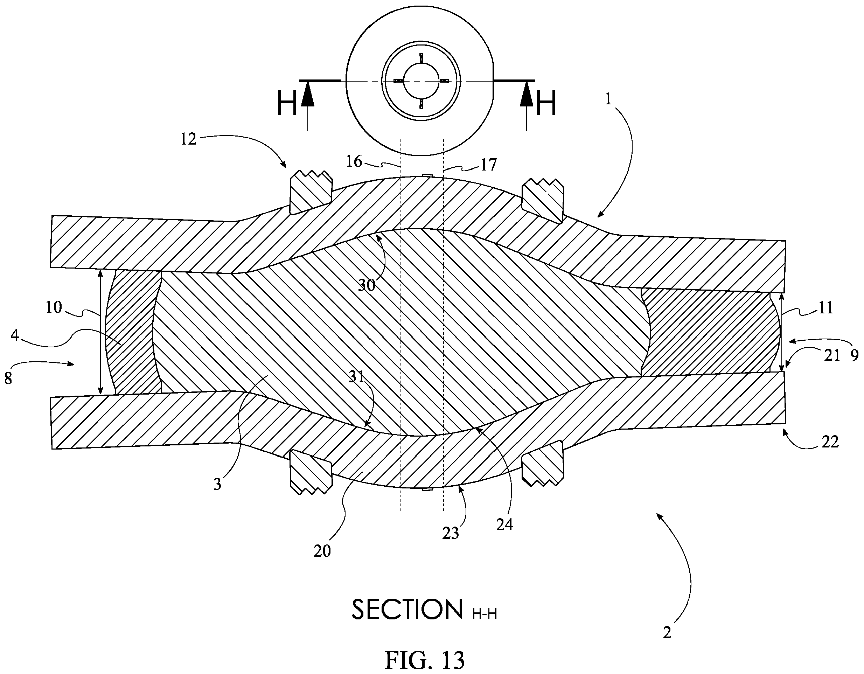

In the preferred embodiment, the inner core **3** is positioned within the outer core **4.** In some embodiments, the inner core **3** is positioned concentrically within the outer core **4,** wherein the inner core **3** comprises an inner core centerline **16** and the outer core **4** comprises an outer core centerline **17,** as shown in FIGS. **2****,** **5****,** **7****,** **8****,** **12****,** **13****,** and **21****.** The inner core centerline **16** and the outer core centerline **17** are positioned coincident with each other in order for the inner core **3** and the outer core **4** to be positioned concentrically with each other, as illustrated in FIG. **5****.** In some embodiments, the inner core **3** and the outer core **4** may be positioned such that they are not exactly concentric, or nonconcentric, with each other, and thus the inner core centerline **16** and the outer core centerline **17** are positioned offset from each other, either through translation or rotation, or both.

In the preferred embodiment, the outer core **4** comprises an inner cavity **40,** and the inner cavity **40** centrally traverses through the outer core **4.** Said central traversement is not intended to mean that the inner cavity **40** is necessarily concentric with the outer perimeter of the outer core **4,** but rather that the inner cavity **40** traverses through the outer core **4** in a generally centralized position relative to the outer perimeter of the outer core **4.** In some embodiments, however, the inner cavity **40** is positioned concentrically within the outer core **4,** such that the inner cavity **40** centrally and axially traverses through the outer core **4.** In some other embodiments, the inner cavity **40** may be positioned nonconcentrically within the outer core **4.** The inner core **3** is positioned within the inner cavity **40** of the outer core **4,** wherein the inner core **3** is sealed by the outer core **4,** the first end plate **1** and the second end plate **2.** In some embodiments, the inner core **3** is positioned concentrically within the inner cavity **40.** In some embodiments, the internal lateral geometry of the inner cavity **40** may be determined by the external lateral geometry of the outer core **4.** In some other embodiments, the inner core **3** may be positioned nonconcentrically within the inner cavity **40.**

In various embodiments, the first end plate **1,** second end plate **2,** inner core **3,** and outer core **4** may be positioned concentrically with each other, wherein each of the first end plate **1,** second end plate **2,** inner core **3,** and outer core **4** comprise a centerline, wherein the centerlines of the first end plate **1,** second end plate **2,** inner core **3,** and outer core **4** are aligned with each other when the first end plate **1,** second end plate **2,** inner core **3,** and outer core **4** are positioned concentrically with each other. In other embodiments, one or more of the first end plate **1,** second end plate **2,** inner core **3,** and outer core **4** may not be positioned concentrically, or nonconcentrically, with each other, wherein the centerlines of one or more of the said components may be positioned offset from each other, either through linear translation or rotation - an offset centerline may be positioned parallel to one or more other centerlines of the aforementioned components, but offset through a linear distance, or an offset centerline may be oriented at an angle offset to one or more other centerlines of the aforementioned components. In some embodiments, such a said centerline may also correspond to a radial axis, or axis of revolution, of the generally radial features of said components.

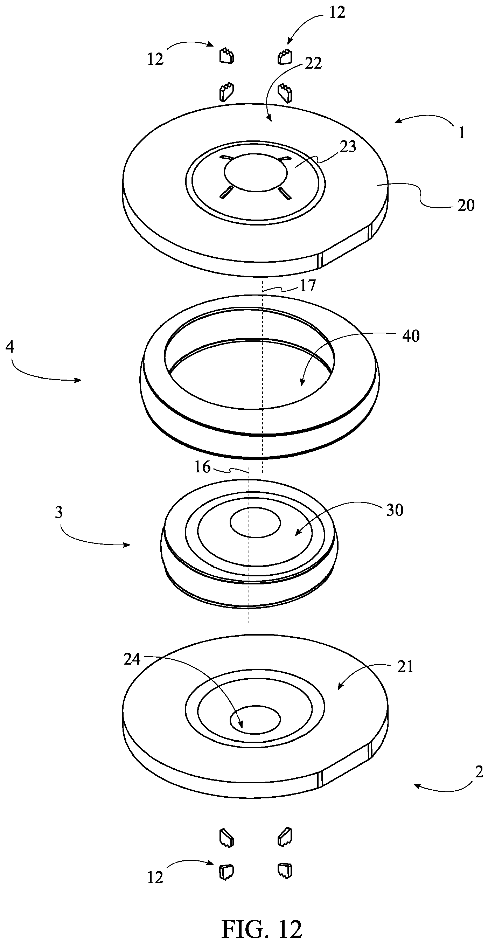

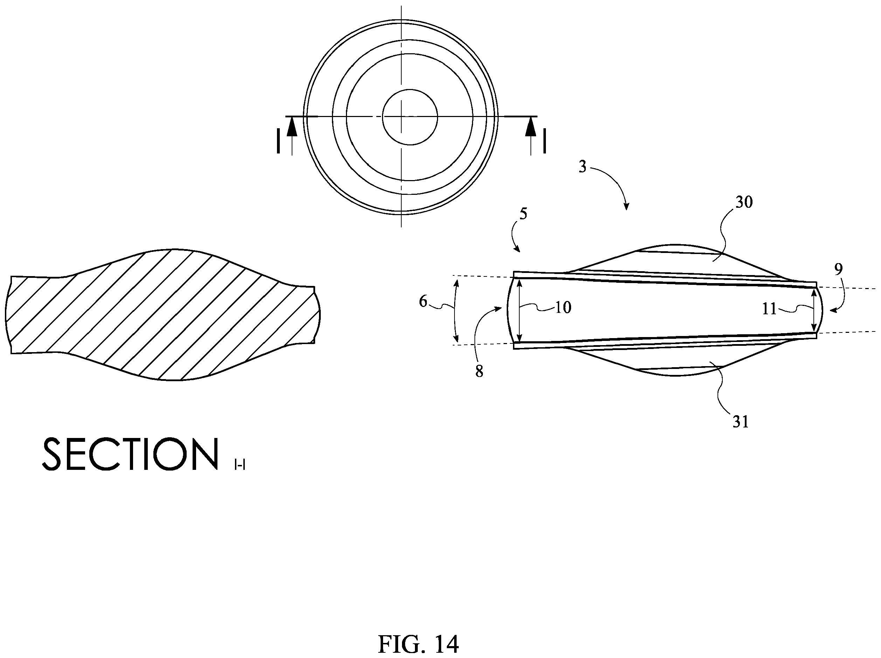

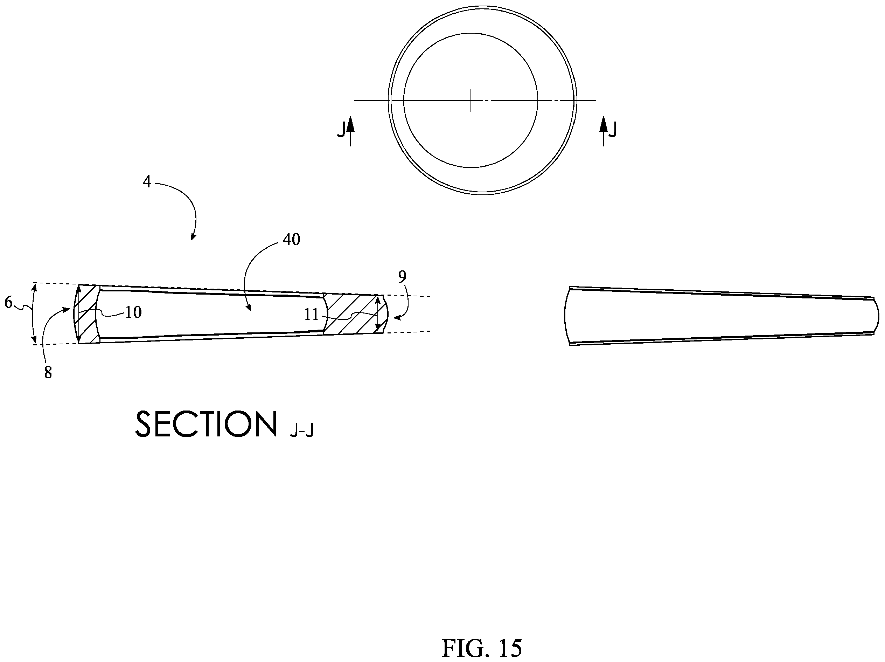

According to the invention, the outer core **4** is connected between the first end plate **1** and the second end plate **2.** In some embodiments, as shown in FIGS. **1-11****,** the outer core **4** is connected between the first end plate **1** and the second end plate **2** through a plurality of interlocking members **5.** In some embodiments (not claimed), the outer core **4** is connected between the first end plate **1** and the second end plate **2** through an adhesive. FIGS. **12-15** and FIGS. **20-24** illustrate examples of such embodiments. The adhesive may be any suitable adhesive appropriate for the intended use of the present invention. The adhesive should be sterile, biodegradable and biocompatible. The adhesive may be based on, derived from, or comprise, but is not limited to, bone cement, polyurethane, acrylates, rubbers, polymers, epoxy resins, phenolic resins, natural adhesives, synthetic adhesives, or any other suitable adhesive substances. Alternatively, the adhesive may be the result of any suitable manufacturing process, as opposed to a distinct adhesive substance.

Thus, the inner core **3** and the outer core **4** are sandwiched by the first end plate **1** and the second end plate **2.** More specifically, as previously mentioned, the outer core **4** comprises the inner cavity **40** that centrally and traverses through the outer core **4,** with the inner core **3** being positioned within the inner cavity **40** of the outer core **4,** such that the inner core **3** is sealed by the outer core **4,** the first end plate **1** and the second end plate **2.**

In the preferred embodiment, the inner core **3** is constructed of a polymeric material. More particularly, in various embodiments, the inner core **3** is preferably constructed of a medical or implant grade polymeric or elastomeric material which may have varying hardness and other physical properties in various embodiments. In some embodiments, the inner core **3** is constructed of a liquid silicon rubber material, wherein the liquid silicon rubber material may have varying hardness properties in different embodiments. In some embodiments, the inner core **3** is constructed of a medical or implant grade silicone elastomer with a hardness ranging from 60 shore A to 90 shore A. In some embodiments, the inner core **3** may be constructed of a liquid silicone rubber (LSR) material. Such material is inert and widely used in medical breast implants. Silicone rubber has the ability to retain its initial shape and mechanical stress under high compression, shear-compression, flexural, torsional, and tensile stresses and has excellent creep properties. In other embodiments, other appropriate materials may be used to manufacture the inner core **3.** The inner core **3** serves as a solid "diaphragm" or cushion that resists and withstands localized compression, shear-compression, torsion, and other forces. In various embodiments, the diameter of the inner core **3** may range from approximately 0.318 centimetres (cm) to 5.72 cm (0.125 inches to 2.25 inches).

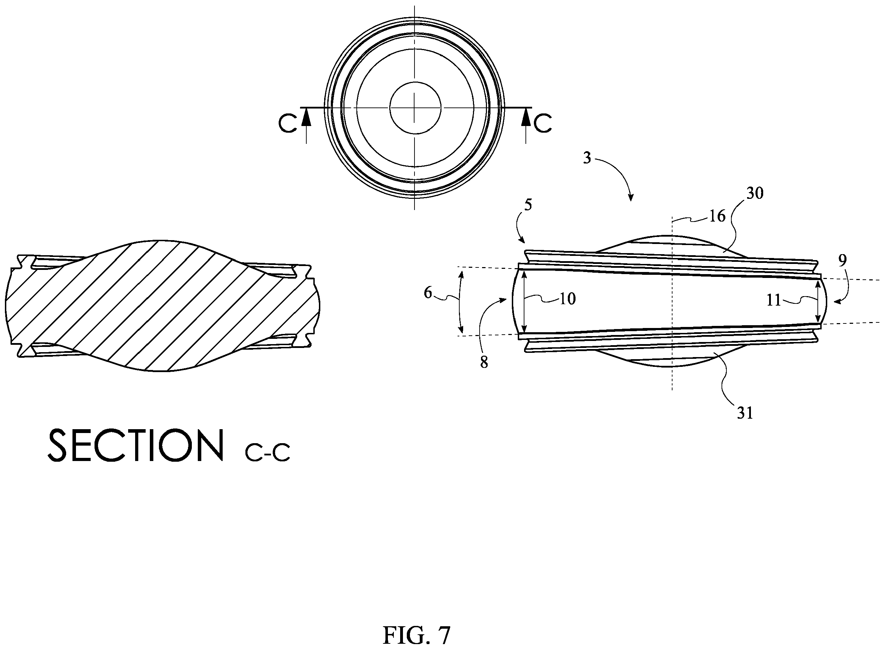

In the preferred embodiment, the inner core **3** comprises a first core convexity **30** and a second core convexity **31,** as shown in FIGS. **5** and **7****.** The first core convexity **30** and the second core convexity **31** are positioned opposite each other along a thickness of the inner core **3,** along the inner core centerline **16.** In some embodiments, the first core convexity **30** and the second core convexity **31** are positioned axially opposite each other along the thickness of the inner core **3,** wherein the first core convexity **30** and the second core convexity **31** are concentrically positioned with the inner core centerline **16.** In other embodiments, the first core convexity **30** and the second core convexity **31** may be positioned offset, nonconcentrically, from the inner core centerline **16.**

The first core convexity **30** and the second core convexity **31** are essentially bulges centrally positioned on the inner core **3** that contribute to the inner core's **3** capabilities to resist and withstand any forces the inner core **3** is subject to while installed in a human spine. The first core convexity **30** and the second core convexity **31** further correspond to and mate with inner concavities on the first end plate **1** and the second end plate **2,** as will be discussed hereinafter. In various embodiments, a convexity angle of the first core convexity **30** and the second core convexity **31** may range from 5 degrees to 60 degrees, and an outer radius of the first core convexity **30** and the second core convexity **31** may range from 0.191 cm to 5 cm (0.075 inches to 2 inches) in various embodiments. In some embodiments, the inner core **3** further comprises a lateral wall with a convex curvature, whose radius may range in various embodiments from 0.160 cm to 5.715 cm (0.063 inches to 2.250 inches).

As previously mentioned, in some embodiments, the inner core **3** comprises an inner core centerline **16** and the outer core **4** comprises an outer core centerline **17.** The inner core centerline **16** and the outer core centerline **17** may be considered in some embodiments to be an axis equidistant from the outer perimeter of the inner core **3** and outer core **4,** respectively, and/or an axis of revolution for other radial features in some embodiments. Additionally or alternatively, in some embodiments, a radial axis may be considered as said axis of revolution, and may be considered as distinct from the inner core centerline **16** and the outer core centerline **17** for the inner core **3** and outer core **4,** respectively. In various embodiments the radial axis may be positioned offset from the inner core centerline **16** and the outer core centerline **17.** In such embodiments, the first core convexity **30** and the second core convexity **31** of the inner core **3** are concentrically positioned about the radial axis of the inner core **3.** Furthermore, the inner cavity **40** of the outer core **4** is positioned concentrically about the radial axis of the outer core **4** in such embodiments. In other embodiments, however, the first core convexity **30** and the second core convexity **31** of the inner core **3** may be nonconcentrically positioned about the radial axis of the inner core **3,** and the inner cavity **40** of the outer core **4** may be positioned nonconcentrically about the radial axis of the outer core **4** in such embodiments.

The outer core **4** acts as a sealing ring for the inner core **3** and provides the necessary motion to the spine once the present invention is implanted in a human body. In the preferred embodiment, the outer core **4** is constructed of a polymeric or elastomeric material with varying hardness and other physical properties in various embodiments.

In various embodiments, the outer core **4** may be constructed of various materials. In the preferred embodiment, the outer core is constructed of a polymeric material. In some embodiments, the outer core is constructed of a liquid silicon rubber material, wherein the liquid silicon rubber material may have varying hardness properties in different embodiments. In some embodiments, the outer core **4** may be constructed of an ultra-high molecular weight polyethylene (UHMWPE) material. In some embodiments, the outer core **4** may be constructed of a medical grade polypropylene (PP) material, though the material of the outer core **4** may vary in different embodiments as desired. In general, it is desired to use a material with superior abrasive and corrosive resistance, high strength, light weight, and low coefficient of friction in the outer core **4.** In various embodiments, the diameter of the outer core **4** may range from 0.445 cm to 6.033 cm (0.175 inches to 2.375 inches), though as previously mentioned, any dimensions listed for the various components of the present invention should not be considered to be limiting and may vary in different embodiments.

In the preferred embodiment, the first end plate **1** and the second end plate **2** are each constructed of a polyether ether ketone (PEEK) material, though the material of the first end plate **1** and the second end plate **2** may vary in different embodiments. PEEK is increasingly employed as a biomaterial for trauma treatments, orthopedic, and spinal implants. It is inherently strong, inert, and biocompatible. Properties that make PEEK a material of choice for the end plates include: modulus similar to bone, reduced stress shielding, artifact-free imaging, and an osteoconductive surface for bone on-growth. Alternatively or additionally, PEEK material can be used in combination with a titanium material or with a titanium plasma spray on the external surfaces of the outer core **4.** The first end plate **1** and the second end plate **2** may be externally treated with a titanium material.

In the preferred embodiment, as shown in FIGS. **1-6****,** the first end plate **1** and the second end plate **2** each comprise a plate body **20,** an inner side **21,** an outer side **22,** a plate convexity **23,** and a concavity **24,** wherein a thickness of the plate body **20** extends between the inner side **21** and the outer side **22.** In various embodiments, the diameter of the plate body **20** may range from 0.953 cm to 6.4 cm (0.375 inches to 2.5 inches), while the thickness may range from 0.079 cm to 0.953 cm (0.031 inches to 0.375 inches).

In the preferred embodiment, the plate body **20** of the first end plate **1** and the plate body **20** of the second end plate **2** are oriented at a specified tilt angle **6** to each other, as illustrated in FIG. **1****.** The specified tilt angle **6** defines a deviation of the plate bodies of the first end plate **1** and second end plate **2** from being oriented parallel to each other. The specified tilt angle **6** may vary in different embodiments, mainly to correspond with different spinal disc types to replace. In various embodiments, the specified tilt angle **6** may range from 0.5 degrees to 15 degrees. In some embodiments, the specified tilt angle **6** may be less than 0.5 degrees. In some embodiments, the specified tilt angle **6** may be 0 degrees, such that the first end plate **1** and the second end plate **2** are oriented parallel to each other. In some embodiments, the specified tilt angle **6** may exceed 15 degrees.

The plate convexity **23** is centrally positioned on the outer side **22** of the plate body **20** for each of the first end plate **1** and the second end plate **2,** similarly, the concavity **24** is centrally positioned on the inner side **21** of the plate body **20** for each of the first end plate **1** and the second end plate **2.** In some embodiments, the thickness of the plate body **20** is constant, and the plate convexity **23** and the concavity **24** are formed through a deviation from the generally flat geometry of the plate body **20,** such that the thickness of the end plates at the plate convexity **23** and concavity **24** is equal to the thickness of the end plates at their perimeter. In other examples, the plate convexity **23** and the concavity **24** of the first end plate **1** and the second end plate **2** may be formed independently of each other (not claimed).

In some embodiments, a fillet may be formed between the plate body **20** and the plate convexity **23** with a radius of, for example, but not limited to, a range from 0.038 cm to 1.3 cm (0.015 inches to 0.5 inches). The fillet serves to reduce any polymeric stress due to vertical localized compression forces on the first and second end plates **2.** The first core convexity **30** is positioned within the concavity **24** of the first end plate **1,** and the second core convexity **31** is positioned within the concavity **24** of the second end plate **2.**

As previously mentioned, in some embodiments, the outer core **4** is connected between the first end plate **1** and the second end plate **2** through the plurality of interlocking members **5.** In some embodiments, the inner core **3** is further connected between the first end plate 1 and the second end plate **2** in the same manner through the plurality of interlocking members **5,** though this is not considered a requirement. More particularly, the inner side **21** of the first end plate **1** is connected to the outer core **4** through the plurality of interlocking members **5,** and the inner side **21** of the second end plate **2** is connected to the outer core **4** opposite the first end plate **1** axially along the inner core **3** through the plurality of interlocking members **5.**

In some embodiments, the first end plate **1** and the second end plate **2** each further comprise an attachment flange **25** and at least one fastener aperture **26,** as shown in FIGS. **1-6****.** The attachment flange **25** may be used to anchor the present invention to adjoining vertebrae through one or more implant screws. The inclusion of the attachment flange **25** in various embodiments will depend on end product requirements. The attachment flange **25** is perpendicularly and perimetrically connected to the plate body **20,** and extends away from the inner side **21,** past the outer side **22** for each of the first end plate **1** and the second end plate **2,** wherein the attachment flange **25** is connected along a flange arc segment of the perimeter of the plate body **20.** Preferably, the attachment flange **25** does not extend past the inner side **21,** though this may vary in different embodiments. The at least one fastener aperture **26** traverses through the attachment flange **25** for each of the first end plate **1** and the second end plate **2.** Each of the at least one fastener aperture **26** may be a counterbore hole, a countersink hole, a through hole, or other types of holes suitable for receiving various fasteners in different embodiments.

Furthermore, in some embodiments, the attachment flange **25** comprises an inner groove **27.** The inner groove **27** traverses into and radially through the attachment flange **25** along the flange arc segment adjacent to the outer side **22** of the plate body **20** and adjacent to a perimeter of the plate body **20** for each of the first end plate **1** and the second end plate **2.** The inner groove **27** serves to provide clearance to the edge of adjoining vertebra where the total disc replacement is being performed to reduce any wear from vertebral edges.

In the preferred embodiment, referring to FIGS. **2** and **5****-7,** the outer core **4** comprises a first plurality of core interlocking members **50** and a second plurality of core interlocking members **51** from the plurality of interlocking members **5,** wherein the first plurality of core interlocking members **50** and the second plurality of core interlocking members **51** are positioned opposite each other along a thickness of the outer core **4.** In some embodiments, the first plurality of core interlocking members **50** and the second plurality of core interlocking members **51** are positioned axially opposite each other along the thickness of the outer core. Further, the first end plate **1** and the second end plate **2** each further comprise a plurality of plate interlocking members **52** from the plurality of interlocking members **5.** The plurality of plate interlocking members **52** is positioned concentrically around the concavity **24** on the inner side **21** of the plate body **20** for each of the first end plate **1** and the second end plate **2.** The first plurality of core interlocking members **50** of the outer core **4** is engaged with the plurality of plate interlocking members **52** of the first end plate **1,** and the second plurality of core interlocking members **51** of the outer core **4** is engaged with the plurality of plate interlocking members **52** of the second end plate **2.**

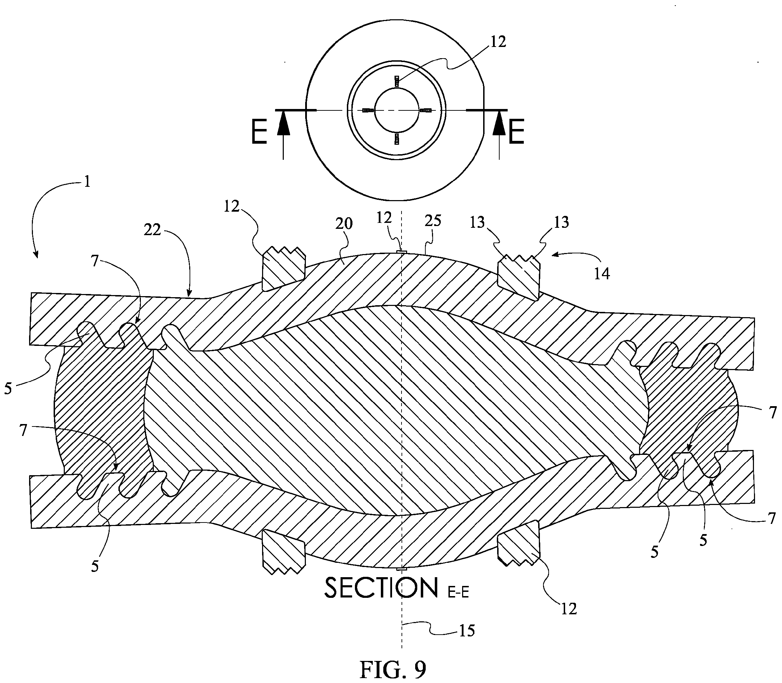

Furthermore, in some embodiments, the present invention further comprises a plurality of interlocking member receiving channels **7,** as shown in FIGS. **2****,** **5****,** **6****,** and **8****-11.** Each of the plurality of interlocking member receiving channels **7** is positioned concentrically with and adjacent to one of the plurality of interlocking members **5,** and each of the plurality of interlocking members **5** is positioned within one of the plurality of interlocking member receiving channels **7.** The plurality of interlocking members **5** and the plurality of interlocking member receiving channels **7** are configured to resist extrusion of the inner core **3** and outer core **4** when the inner core **3** and outer core **4** are subject to external forces. The specific configuration and shape of the plurality of interlocking members **5** and the plurality of interlocking member receiving channels **7** may vary in different embodiments. For example, in some embodiments, the plurality of interlocking members **5** and the plurality of interlocking member receiving channels **7** have a dovetail mating configuration as shown in FIGS. **2** and **5****-8,** wherein a cross section width of each of the plurality of interlocking members **5** increases with distance away from the axial center of the outer core **4,** and wherein each of the plurality of interlocking member receiving channels **7** is shaped inversely to the plurality of interlocking members **5** to accommodate them. In some embodiments, a cross section of each of the plurality of interlocking members **5** resembles a "tooth", having an angled protrusion with a circular element at the end, wherein the cross section of the interlocking member receiving channels **7** would have correspondingly negative geometry to receive the tooth. In either case, the distal geometry of the interlocking members **5** has a greater radial width than the remainder of the interlocking members **5,** facilitating securement of the interlocking members **5** within the interlocking member receiving channels **7.** Alternatively, in some embodiments, the interlocking members **5** and receiving channels **7** may not vary in thickness, as shown in FIG. **9****.**

More particularly, FIGS. **9-11** illustrate an alternative embodiment wherein each of the plurality of interlocking members **5** is oriented at a specified anti-extrusion angle **53** in order to adequately resist extrusion of the inner core **3** and/or outer core **4** as previously described. In such an embodiment. More particularly, each of the first plurality of core interlocking members **50,** the second plurality of core interlocking members **51,** and the plurality of plate interlocking members **52** is oriented at the specified anti-extrusion angle **53.** The specified anti-extrusion angle **53** may be defined in various ways, but herein the specified anti-extrusion angle **53** is defined in relation to a central axis **15** of the present invention. The central axis **15** may be considered to be an axis about which the radial features of the present invention, such as, but not limited to the inner core **3** and the outer core **4,** and the plate body **20** are positioned concentrically about. Separate central axes may be defined as appropriate for the inner core **3,**outer core **4,** and plate body **20** in order to account for any angular discrepancies due to the specified tilt angle **6.** The previously discussed inner core centerline **16** and outer core centerline **17** may, in various embodiments, be the same as or distinct from the central axis **15.**

It may be understood herein that due to the specified tile angle **6,** true central axes of the plate body **20,** inner core **3,** and/or outer core **4** may not be positioned exactly coincidental with each other. However, that is considered to be the case herein for the sake of simplicity.

It is important to define herein that the specified anti-extrusion angle **53** should be oriented radially outward from the central axis, such that an imaginary line extending outward from any given member of the first plurality of core interlocking members **50** or the second plurality of core interlocking members **51** does not intersect with the central axis. This is important because an inwardly-oriented anti-extrusion angle **53** would not be effective or as effective and may not provide effective interlock as an outwardly-oriented anti-extrusion angle.

As previously mentioned, the plurality of interlocking members **5** and the plurality of interlocking member receiving channels **7** function primarily to secure the inner core **3** to the first end plate **1** and the second end plate **2,** but also secondarily to resist extrusion of the outer core **4** (and inner core **3,** in applicable embodiments) when the present invention is subjected to external forces, compressive forces in particular. When the present invention is subjected to an axial compressive force, the inner core **3** and outer core **4** will tend to deform a certain amount in compression axially and in expansion laterally. Thus, is it a concern that subject to such forces, portions of the inner core **3** and outer core **4** will "extrude" out of their designated positions and potentially become misaligned or damaged. The plurality of interlocking member receiving channels **7** may function to provide some space to accommodate such extrusion, and moreover the physical interlocking between the interlocking members **5** and interlocking member receiving channels **7** prevents the inner core **3** and outer core **4** from becoming dislodged from their positions relative to the first end plate **1** and the second end plate **2.** The quantity of both the plurality of interlocking members **5** and the plurality of interlocking member receiving channels **7** may vary in different embodiments, from 1 to 9, for example, though any quantity of interlocking members **5** and interlocking member receiving channels **7** may be included as desired in various embodiments.

As previously mentioned, in various embodiments, the first end plate **1** and the second end plate **2** may be oriented at a specified tilt angle **6** to each other in order to imitate the geometry of a spinal disc to be replaced by the present invention. As such, the plate body **20** of the first end plate **1,** the plate body **20** of the second end plate **2,** the inner core **3** and the outer core **4** may be understood to extend in a longitudinal direction between a proximal end **8** and a distal end **9.** The proximal end **8** and the distal end **9** are positioned diametrically opposite each other for each of the plate body **20,** the inner core **3,** and the outer core **4.** The proximal ends **8** are defined herein to be radially aligned with each other and the distal ends **9** are radially aligned with each other for each of the plate bodies of the first end plate **1** and the second end plate **2,** the inner core **3,** and the outer core **4.** Thus, in some embodiments, the specified tilt angle **6** is defined in a plane coincident with the proximal ends **8** and the distal ends **9.** In some embodiments comprising the flange attachment, the flange attachment is positioned at the proximal end **8** for each of the first end plate **1** and the second end plate **2.** In various embodiments, the orientation and alignment of the specified tilt angle **6** may vary however, and should not be considered to be limited to the foregoing description.

Moreover, referring to FIGS. **7-8****,** in some embodiments, the specified tilt angle **6** may be realized through a diametrical difference in thickness of the inner core **3** and the outer core **4,** so that the axial outer ends of the inner core **3** and outer core **4** are oriented at the specified tilt angle **6** to each other, and thus the first end plate **1** and the second end plate **2,** being generally flat, are oriented at the specified tilt angle **6** to each other as a result. As such, a proximal thickness **10** and a distal thickness **11** of both the inner core **3** and the outer core **4** may be defined, wherein the proximal thickness **10** of the outer core **4** is the thickness of the outer core **4** at the proximal end **8** of the outer core **4,** the distal thickness **11** of the outer core **4** is the thickness of the outer core **4** at the distal end **9** of the outer core **4,** the proximal thickness **10** of the inner core **3** is the thickness of the inner core **3** at the proximal end **8** of the inner core **3,** and the distal thickness **11** of the inner core **3** is the thickness of the inner core **3** at the distal end **9** of the inner core **3.**

Thus, in some embodiments, the proximal thickness **10** of the inner core **3** is greater than the distal thickness **11** of the inner core **3,** and the proximal thickness **10** of the outer core **4** is greater than the distal thickness **11** of the outer core **4.** Thus, the specified tilt angle **6** may be determined in some embodiments by the difference between the proximal thicknesses **10** and the distal thicknesses **11.** In other embodiments, the specified tilt angle **6** may be determined through other means; for example, the thickness of the inner core **3** and outer core **4** may be constant, while the thickness of the first end plate **1** and second end plate **2** may vary instead.

In some embodiments of the present invention, as shown in FIG. **9****,** as an alternative to the attachment flange **25,** the first end plate **1** and the second end plate **2** further comprise a plurality of anchoring protrusions **12.** The plurality of anchoring protrusions **12** serve as an alternative means of mounting the first end plate **1** and the second end plate **2** to adjacent vertebrae. The anchoring protrusions **12** are connected to the outer side **22** and are preferably oriented perpendicular to the plate body **20.** The anchoring protrusions **12** are distributed around the outer side **22** in any desirable configuration, such as, but not limited to, four anchoring protrusions positioned in a concentric 90 degree pattern around the plate convexity **25.** Furthermore, each of the anchoring protrusions **12** may comprise a plurality of teeth **13** positioned at a distal end **14** of the anchoring protrusions **12.**

The components of the present invention may be manufactured through any desirable manufacturing process, such as, but not limited to, 3D printing, CNC machining, injection molding, compression bolding, or other manufacturing processes. The inner core **3** is preferably injection molded through an insert molding process where the first end plate **1** and the second end plate **2** serve as inserts. Alternatively, the inner core **3** can be produced independently through an injection molding, compression molding, or 3D printing process and is subsequently assembled with the first end plate **1** and the second end plate **2.** The outer core **4** is preferably either injection molded or compression molded using an insert molding process where an assembly of the first end plate **1,** second end plate **2,** and inner core **3** serve as inserts. Alternatively, the outer core **4** can be produced independently through injection molding, compression molding, or 3D printing and subsequently assembled with the first end plate **1,** second end plate **2,** and inner core **3.** Furthermore, in the preferred embodiment, at every stage of assembly of the present invention, the external surfaces of the various components of the present invention are treated to increase surface bonding to achieve sufficient covalent, cohesive and/or adhesive bonds.

FIGS. **12-15** show a second alternate embodiment (not claimed) wherein the inner cavity **40** of the outer core **4** is positioned offset from concentric with the outer core centerline **17,** and the first core convexity **30** and second core convexity **31** of the inner core **3** are positioned offset from concentric with the inner core centerline **16.** Furthermore, the inner core centerline **16** and the outer core centerline **17** are also offset from each other. This geometric arrangement may have various advantages in different use cases, such as facilitating conformity to a patient's vertebrae. The second alternate embodiment additionally utilizes the previously discussed adhesive to bond the outer core **4** to the first end plate **1** and the second end plate **2.**

FIGS. **16-19** show a third alternate embodiment, wherein the peripheral geometry of the inner core **3** and outer core **4** are generally rectilinear. This geometric arrangement may also have similar various advantages in different use cases or implantation scenarios, such as facilitating ease of insertion of the present invention between vertebrae.

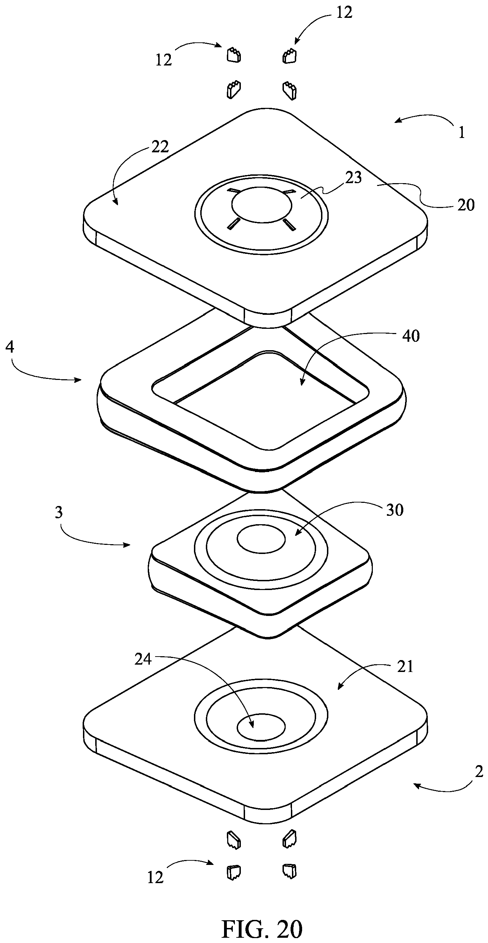

FIGS. **20-24** show a fourth alternate embodiment (not claimed), wherein the peripheral geometry of the inner core **3** and outer core **4** are generally rectilinear, as with the third alternate embodiment, while the outer core **4** is connected between the first end plate **1** and the second end plate **2** through the adhesive, as with the second alternate embodiment. Different embodiments comprising various combinations of the aforementioned features may be considered to be more or less advantageous in various situations, and the present invention should not be considered to be strictly limited to any of the specifically described embodiments herein.

## Claims

1. A motion preserving spinal implant for total disc replacement comprising:
a first end plate (1), wherein the first end plate (1) comprises a plate body (20), a plate convexity (23) and plate concavity (24) disposed on the plate body (20);
wherein the plate body (20) of first end plate (1) further comprises an inner side (21) and outside side (22);
wherein plate convexity (23) is centrally positioned on the outside side (22) of the first end plate (1);
wherein plate concavity (24) is centrally positioned on the inner side (21) of the first end plate (1);
wherein the convexity of plate convexity (23) is equal to the concavity of plate concavity (24);
a second end plate (2), wherein the second end plate (2) comprises a plate body (20), a plate convexity (23) and plate concavity (24) disposed on the plate body (20);
wherein the plate body (20) of second end plate (2) further comprises an inner side (21) and outside side (22);
wherein plate convexity (23) is centrally positioned on the outside side (22) of the second end plate (2);
wherein plate concavity (24) is centrally positioned on the inner side (21) of the second end plate (2);
wherein the convexity of plate convexity (23) is equal to the concavity of plate concavity (24);
an outer core (4) having a body and an inner cavity (40) traversing through the outer core, wherein the outer core (4) is disposed between the first end plate (1) and the second end plate (2);
wherein a plurality of core interlocking members (50) disposed on the body of outer core (4) engages a plurality of plate interlocking members (52) disposed on the inner side (21) of first end plate (1);
wherein a plurality of core interlocking members (51) disposed on the body of outer core (4) engages a plurality of plate interlocking members (52) disposed on the inner side (21) of second end plate (2);
an inner core (3) comprising a first core convexity (30) and a second core convexity (31) positioned opposite each other along a thickness of the inner core;
wherein the inner core (3) is disposed in the inner cavity (40), whereby the first core convexity (30) is disposed within the concavity (24) of the first end plate (1) and the second core convexity (31) is disposed within the concavity (24) of the second end plate (2);
wherein the inner core (3) is sealed by the outer core (4), the first end plate (1), and the second end plate (2).

2. The motion preserving spinal implant for total disc replacement as claimed in claim 1 comprising:
the inner cavity centrally and axially traversing through the outer core (4); and
the inner core (3) being concentrically positioned within the inner cavity.

3. The motion preserving spinal implant for total disc replacement as claimed in claim 1, wherein the first end plate (1), the second end plate (2), the inner core (3), and the outer core (4) each have rectilinear peripheral geometry.

4. The motion preserving spinal implant for total disc replacement as claimed in claim 1 comprising:
the inner core (3) and the outer core (4) each comprising a radial axis and a central axis;
the radial axis being positioned offset from the central axis for each of the inner core (3) and the outer core (4); and
the first core convexity and the second core convexity of the inner core (3) being concentrically positioned about the radial axis of the inner core (3).

5. The motion preserving spinal implant for total disc replacement as claimed in claim 1, wherein the outer core (4) is connected between the first end plate (1) and the second end plate (2) through an adhesive.

6. The motion preserving spinal implant for total disc replacement as claimed in claim 1, wherein the plate body (20) of the first end plate (1) and the plate body (20) of the second end plate (2) are oriented at a specified tilt angle to each other, wherein the specified tilt angle defines a deviation of the plate body (20) of the first end plate (1) and the plate body (20) of the second end plate (2) from being oriented parallel to each other; and wherein the specified tilt angle is within a range of 0 to 15 degrees.

7. The motion preserving spinal implant for total disc replacement as claimed in claim 1, wherein the inner core (3) is constructed of a polymeric material.

8. The motion preserving spinal implant for total disc replacement as claimed in claim 1, wherein the first end plate (1) and the second end plate (2) are constructed of a polyether ether ketone (PEEK) material.

9. The motion preserving spinal implant for total disc replacement as claimed in claim 1, wherein the inner core (3) is constructed of a liquid silicon rubber with varying hardness.

10. The motion preserving spinal implant for total disc replacement as claimed in claim 1, wherein the outer core (4) is constructed of a liquid silicon rubber with varying hardness.

11. The motion preserving spinal implant for total disc replacement as claimed in claim 1, wherein the outer core (4) is constructed of a polymeric material.

12. The motion preserving spinal implant for total disc replacement as claimed in claim 1 comprising:
the first end plate (1) and the second end plate (2) each further comprising an attachment flange (25) and at least one fastener aperture (26);
the attachment flange (25) being perpendicularly and perimetrically connected to the plate body and extending away from the inner side, past the outer side for each of the first end plate (1) and the second end plate (2), wherein the attachment flange (25) is connected along a flange arc segment of the perimeter of the plate body; and
the at least one fastener aperture (26) traversing through the attachment flange (25) for each of the first end plate (1) and the second end plate (2).

13. The motion preserving spinal implant for total disc replacement as claimed in claim 12 comprising:
the attachment flange (25) comprising an inner groove (27); and
the inner groove (27) traversing radially through the attachment flange (25) along the flange arc segment adjacent to the outer side and adjacent to a perimeter of the plate body for each of the first end plate (1) and the second end plate (2).

14. The motion preserving spinal implant for total disc replacement as claimed in claim 1 comprising:
the outer core (4) comprising a first plurality of core interlocking members and a second plurality of core interlocking members from the plurality of interlocking members, wherein the first plurality of core interlocking members and the second plurality of core interlocking members are positioned opposite each other along a thickness of the outer core (4);
the first end plate (1) and the second end plate (2) each further comprising a plurality of plate interlocking members from the plurality of interlocking members;
the plurality of plate interlocking members being positioned concentrically around the concavity on the inner side of the plate body for each of the first end plate (1) and the second end plate (2);
the first plurality of core interlocking members of the outer core (4) being engaged with the plurality of plate interlocking members of the first end plate (1); and
the second plurality of core interlocking members of the outer core (4) being engaged with the plurality of plate interlocking members of the second end plate (2).

15. The motion preserving spinal implant for total disc replacement as claimed in claim 14 comprising:
a plurality of interlocking member receiving channels (7);
each of the plurality of interlocking member receiving channels (7) being positioned concentrically with and adjacent to one of the plurality of interlocking members; and
each of the plurality of interlocking members being positioned within one of the plurality of interlocking member receiving channels (7),
wherein the plurality of interlocking members and the plurality of interlocking member receiving channels (7) are configured to resist extrusion of the inner core (3) and outer core (4) when the inner core (3) and outer core (4) are subject to external forces.

16. The motion preserving spinal implant for total disc replacement as claimed in claim 6 comprising:
the plate body of the first end plate (1), the plate body of the second end plate (2), the inner core (3) and the outer core (4) each extending longitudinally between a proximal end and a distal end,
wherein the proximal end and the distal end are positioned diametrically opposite each other for each of the plate body, the inner core (3), and the outer core (4),
wherein the proximal ends are radially aligned with each other and the distal ends are radially aligned with each other for each of the plate bodies of the first end plate (1) and second end plate (2), the inner core (3), and the outer core (4), and
wherein the specified tilt angle is defined in a plane coincident with the proximal ends and the distal ends.

17. The motion preserving spinal implant for total disc replacement as claimed in claim 16 comprising:
the first end plate (1) and the second end plate (2) each further comprising a flange attachment; and
the flange attachment being positioned at the proximal end for each of the first end plate (1) and the second end plate (2).

18. The motion preserving spinal implant for total disc replacement as claimed in claim 16 comprising:
a proximal thickness of the inner core (3) at the proximal end of the inner core (3) being greater than a distal thickness of the inner core (3) at the distal end of the inner core (3); and
a proximal thickness of the outer core (4) at the proximal end of the outer core (4) being greater than a distal thickness of the outer core (4) at the distal end of the outer core (4), wherein the specified tilt angle is determined by the difference between the proximal thicknesses and the distal thicknesses.

## Patentansprüche

1. Bewegungserhaltendes Wirbelsäulenimplantat für Bandscheibentotalersatz, umfassend:
eine erste Endplatte (1), wobei die erste Endplatte (1) einen Plattenkörper (20), eine Plattenkonvexität (23) und eine Plattenkonkavität (24), die auf dem Plattenkörper (20) angeordnet sind, umfasst;
wobei der Plattenkörper (20) der ersten Endplatte (1) weiterhin eine Innenseite (21) und eine Außenseite (22) umfasst;
wobei die Plattenkonvexität (23) zentral auf der Außenseite (22) der ersten Endplatte (1) positioniert ist;
wobei die Plattenkonkavität (24) zentral auf der Innenseite (21) der ersten Endplatte (1) positioniert ist;
wobei die Konvexität der Plattenkonvexität (23) gleich der Konkavität der Plattenkonkavität (24) ist;
eine zweite Endplatte (2), wobei die zweite Endplatte (2) einen Plattenkörper (20), eine Plattenkonvexität (23) und eine Plattenkonkavität (24), die auf dem Plattenkörper (20) angeordnet sind, umfasst;
wobei der Plattenkörper (20) der zweiten Endplatte (2) weiterhin eine Innenseite (21) und eine Außenseite (22) umfasst;
wobei die Plattenkonvexität (23) zentral auf der Außenseite (22) der zweiten Endplatte (2) positioniert ist;
wobei die Konvexität der Plattenkonvexität (23) gleich der Konkavität der Plattenkonkavität (24) ist;
einen äußeren Kern (4) mit einem Körper und einen inneren Hohlraum (40), der durch den äußeren Kern hindurchtritt, wobei der äußere Kern (4) zwischen der ersten Endplatte (1) und der zweiten Endplatte (2) angeordnet ist;
wobei eine Vielzahl von ineinandergreifenden Kernelementen (50), die auf dem Körper des äußeren Kerns (4) angeordnet sind, eine Vielzahl von ineinandergreifenden Plattenelementen (52), die auf der Innenseite (21) der ersten Endplatte (1) angeordnet sind, in Eingriff nimmt;
wobei eine Vielzahl von ineinandergreifenden Kernelementen (51), die auf dem Körper des äußeren Kerns (4) angeordnet sind, eine Vielzahl von ineinandergreifenden Plattenelementen (52), die auf der Innenseite (21) der zweiten Endplatte (2) angeordnet sind, in Eingriff nimmt;
einen inneren Kern (3), der eine erste Kernkonvexität (30) und eine zweite Kernkonvexität (31) umfasst, die entgegengesetzt zueinander entlang einer Dicke des inneren Kerns positioniert sind,
wobei der innere Kern (3) in dem inneren Hohlraum (40) angeordnet ist, wodurch die erste Kernkonvexität (30) innerhalb der Konkavität (24) der ersten Endplatte (1) angeordnet ist und die zweite Kernkonvexität (31) innerhalb der Konkavität (24) der zweiten Endplatte (2) angeordnet ist;
wobei der innere Kern (3) durch den äußeren Kern (4), die erste Endplatte (1) und die zweite Endplatte (2) versiegelt ist.

2. Bewegungserhaltendes Wirbelsäulenimplantat für Bandscheibentotalersatz nach Anspruch 1, umfassend:
dass der innere Hohlraum durch den äußeren Kern (4) zentral und axial hindurchtritt; und
der innere Kern (3) konzentrisch innerhalb des inneren Hohlraums positioniert ist.

3. Bewegungserhaltendes Wirbelsäulenimplantat für Bandscheibentotalersatz nach Anspruch 1, wobei die erste Endplatte (1), die zweite Endplatte (2), der innere Kern (3) und der äußere Kern (4) jeweils eine geradlinige Randgeometrie aufweisen.

4. Bewegungserhaltendes Wirbelsäulenimplantat für Bandscheibentotalersatz nach Anspruch 1, umfassend:
der innere Kern (3) und der äußere Kern (4) jeweils eine radiale Achse und eine zentrale Achse umfassen;
wobei die radiale Achse für jeden von dem inneren Kern (3) und dem äußeren Kern (4) versetzt von der zentralen Achse positioniert ist; und
die erste Kernkonvexität und die zweite Kernkonvexität des inneren Kerns (3) konzentrisch um die radiale Achse des inneren Kerns (3) positioniert sind.

5. Bewegungserhaltendes Wirbelsäulenimplantat für Bandscheibentotalersatz nach Anspruch 1, wobei der äußere Kern (4) zwischen der ersten Endplatte (1) und der zweiten Endplatte (2) durch einen Klebstoff verbunden ist.

6. Bewegungserhaltendes Wirbelsäulenimplantat für Bandscheibentotalersatz nach Anspruch 1, wobei der Plattenkörper (20) der ersten Endplatte (1) und der Plattenkörper (20) der zweiten Endplatte (2) in einem spezifizierten Neigungswinkel zueinander ausgerichtet sind, wobei der spezifizierte Neigungswinkel eine Abweichung des Plattenkörpers (20) der ersten Endplatte (1) und des Plattenkörpers (20) der zweiten Endplatte (2) von einer parallelen Ausrichtung zueinander definiert; und wobei der spezifizierte Neigungswinkel innerhalb eines Bereichs von 0 bis 15 Grad liegt.

7. Bewegungserhaltendes Wirbelsäulenimplantat für Bandscheibentotalersatz nach Anspruch 1, wobei der innere Kern (3) aus einem polymeren Material konstruiert ist.

8. Bewegungserhaltendes Wirbelsäulenimplantat für Bandscheibentotalersatz nach Anspruch 1, wobei die erste Endplatte (1) und die zweite Endplatte (2) aus einem Polyetheretherketon-Material (PEEK-Material) konstruiert sind.

9. Bewegungserhaltendes Wirbelsäulenimplantat für Bandscheibentotalersatz nach Anspruch 1, wobei der innere Kern (3) aus einem flüssigen Siliziumkautschuk mit variierender Härte konstruiert ist.

10. Bewegungserhaltendes Wirbelsäulenimplantat für Bandscheibentotalersatz nach Anspruch 1, wobei der äußere Kern (4) aus einem flüssigen Siliziumkautschuk mit variierender Härte konstruiert ist.

11. Bewegungserhaltendes Wirbelsäulenimplantat für Bandscheibentotalersatz nach Anspruch 1, wobei der äußere Kern (4) aus einem polymeren Material konstruiert ist.

12. Bewegungserhaltendes Wirbelsäulenimplantat für Bandscheibentotalersatz nach Anspruch 1, umfassend:
dass die erste Endplatte (1) und die zweite Endplatte (2) jeweils weiterhin einen Anbringungsflansch (25) und mindestens eine Befestigungselementöffnung (26) umfassen;
wobei der Anbringungsflansch (25) senkrecht und umfänglich mit dem Plattenkörper verbunden ist und sich für jede von der ersten Endplatte (1) und der zweiten Endplatte (2) von der Innenseite weg, an der Außenseite vorbei erstreckt,
wobei der Anbringungsflansch (25) entlang einem Flanschbogensegment des Umfangs des Plattenkörpers verbunden ist; und
die mindestens eine Befestigungselementöffnung (26) für jede von der ersten Endplatte (1) und der zweiten Endplatte (2) durch den Anbringungsflansch (25) hindurchtritt.

13. Bewegungserhaltendes Wirbelsäulenimplantat für Bandscheibentotalersatz nach Anspruch 12, umfassend:
dass der Anbringungsflansch (25) eine innere Nut (27) umfasst; und
wobei die innere Nut (27) für jede von der ersten Endplatte (1) und der zweiten Endplatte (2) radial durch den Anbringungsflansch (25) entlang dem Flanschbogensegment angrenzend an die Außenseite und angrenzend an einen Umfang des Plattenkörpers hindurchtritt.

14. Bewegungserhaltendes Wirbelsäulenimplantat für Bandscheibentotalersatz nach Anspruch 1, umfassend:
dass der äußere Kern (4) eine erste Vielzahl von ineinandergreifenden Kernelementen und eine zweite Vielzahl von ineinandergreifenden Kernelementen von der Vielzahl von ineinandergreifenden Elementen umfasst, wobei die erste Vielzahl von ineinandergreifenden Kernelementen und eine zweite Vielzahl von ineinandergreifenden Kernelementen entgegengesetzt zueinander entlang einer Dicke des äußeren Kerns (4) positioniert sind,
die erste Endplatte (1) und die zweite Endplatte (2) jeweils weiterhin eine Vielzahl von ineinandergreifenden Plattenelementen von der Vielzahl von ineinandergreifenden Elementen umfassen;
wobei die Vielzahl von ineinandergreifenden Plattenelementen für jede von der ersten Endplatte (1) und der zweiten Endplatte (2) konzentrisch um die Konkavität auf der Innenseite des Plattenkörpers positioniert ist;
die erste Vielzahl von ineinandergreifenden Kernelementen des äußeren Kerns (4) mit der Vielzahl von ineinandergreifenden Plattenelementen der ersten Endplatte (1) im Eingriff steht und
die zweite Vielzahl von ineinandergreifenden Kernelementen des äußeren Kerns (4) mit der Vielzahl von ineinandergreifenden Plattenelementen der zweiten Endplatte (2) im Eingriff steht.

15. Bewegungserhaltendes Wirbelsäulenimplantat für Bandscheibentotalersatz nach Anspruch 14, umfassend:
eine Vielzahl von Kanälen (7) zur Aufnahme von ineinandergreifenden Elementen;
wobei jeder von der Vielzahl von Kanälen (7) zur Aufnahme von ineinandergreifenden Elementen konzentrisch mit einem und angrenzend an eines von der Vielzahl von ineinandergreifenden Elementen positioniert ist; und
jedes von der Vielzahl von ineinandergreifenden Elementen innerhalb eines von der Vielzahl von Kanälen (7) zur Aufnahme von ineinandergreifenden Elementen positioniert ist,
wobei die Vielzahl von ineinandergreifenden Elementen und die Vielzahl von Kanälen (7) zur Aufnahme von ineinandergreifenden Elementen dazu konfiguriert sind, einer Extrusion des inneren Kerns (3) und des äußeren Kerns (4) zu widerstehen, wenn der innere Kern (3) und der äußere Kern (4) externen Kräften unterliegen.

16. Bewegungserhaltendes Wirbelsäulenimplantat für Bandscheibentotalersatz nach Anspruch 6, umfassend:
dass der Plattenkörper der ersten Endplatte (1), der Plattenkörper der zweiten Endplatte (2), der innere Kern (3) und der äußere Kern (4) sich jeweils längs zwischen einem proximalen Ende und einem distalen Ende erstrecken,
wobei das proximale Ende und das distale Ende für jeden von dem Plattenkörper, dem inneren Kern (3) und dem äußeren Kern (4) genau entgegengesetzt zueinander positioniert sind,
wobei die proximalen Enden radial aufeinander ausgerichtet sind und die distalen Enden für jeden von den Plattenkörpern der ersten Endplatte (1) und der zweiten Endplatte (2), den inneren Kern (3) und den äußeren Kern (4) radial aufeinander ausgerichtet sind, und
wobei der spezifizierte Neigungswinkel in einer Ebene definiert ist, die mit den proximalen Enden und den distalen Enden zusammenfällt.

17. Bewegungserhaltendes Wirbelsäulenimplantat für Bandscheibentotalersatz nach Anspruch 16, umfassend:
dass die erste Endplatte (1) und die zweite Endplatte (2) jeweils weiterhin eine Flanschanbringung umfassen; und
wobei die Flanschanbringung für jede von der ersten Endplatte (1) und der zweiten Endplatte (2) am proximalen Ende positioniert ist.

18. Bewegungserhaltendes Wirbelsäulenimplantat für Bandscheibentotalersatz nach Anspruch 16, umfassend:
dass eine proximale Dicke des inneren Kerns (3) am proximalen Ende des inneren Kerns (3) größer als eine distale Dicke des inneren Kerns (3) am distalen Ende des inneren Kerns (3) ist; und
eine proximale Dicke des äußeren Kerns (4) am proximalen Ende des äußeren Kerns (4) größer als eine distale Dicke des äußeren Kerns (4) am distalen Ende des äußeren Kerns (4) ist, wobei der spezifizierte Neigungswinkel durch den Unterschied zwischen den proximalen Dicken und den distalen Dicken definiert ist.

## Revendications

1. Un implant rachidien préservant le mouvement pour le remplacement total du disque comprenant :
une première plaque d'extrémité (1), ladite première plaque d'extrémité (1) comprenant un corps de plaque (20), une convexité de plaque (23) et une concavité de plaque (24) disposées sur le corps de plaque (20) ; dans lequel le corps de plaque (20) de la première plaque d'extrémité (1) comprend en outre un côté intérieur (21) et un côté extérieur (22) ;
dans lequel la convexité de la plaque (23) est positionnée au centre sur le côté extérieur (22) de la première plaque d'extrémité (1) ;
dans lequel la concavité de plaque (24) est positionnée au centralement sur le côté interne (21) de la première plaque d'extrémité (1) ;
dans lequel la convexité de la convexité de la plaque (23) est égale à la concavité de la concavité de la plaque (24) ;
une seconde plaque d'extrémité (2), ladite seconde plaque d'extrémité (2) comprenant un corps de plaque (20), une convexité de plaque (23) et une concavité de plaque (24) disposées sur le corps de plaque (20) ;
dans lequel le corps de plaque (20) de la seconde plaque d'extrémité (2) comprend en outre un côté intérieur (21) et un côté extérieur (22) ;
dans lequel la convexité de la plaque (23) est positionnée centralement sur le côté extérieur (22) de la seconde plaque d'extrémité (2) ;
dans lequel la concavité de la plaque (24) est positionnée centralement sur le côté interne (21) de la seconde plaque d'extrémité (2) ;
dans lequel la convexité de la convexité de la plaque (23) est égale à la concavité de la concavité de la plaque (24) ;
un noyau externe (4) ayant un corps et une cavité interne (40) traversant le noyau externe, dans lequel le noyau externe (4) étant disposé entre la première plaque d'extrémité (1) et la seconde plaque d'extrémité (2) ;
dans lequel une pluralité d'éléments à emboîtement de noyau (50) disposés sur le corps du noyau externe (4) engage une pluralité d'éléments à emboîtement de plaque (52) disposés sur le côté interne (21) de la première plaque d'extrémité (1) ;
dans lequel une pluralité d'éléments à emboîtement de noyau (51) disposés sur le corps du noyau externe (4) engage une pluralité d'éléments à emboîtement de plaque (52) disposés sur le côté interne (21) de la seconde plaque d'extrémité (2) ;
un noyau interne (3) comprenant une première convexité de noyau (30) et une seconde convexité de noyau (31) positionnées l'une en face de l'autre le long d'une épaisseur du noyau interne ;
dans lequel le noyau interne (3) est disposé dans la cavité interne (40), la première convexité de noyau (30) étant disposée à l'intérieur de la concavité (24) de la première plaque d'extrémité (1) et la seconde convexité de noyau (31) étant disposé à l'intérieur de la concavité (24) de la seconde plaque d'extrémité (2) ;
dans lequel le noyau interne (3) étant scellé par le noyau externe (4), la première plaque d'extrémité (1) et la seconde plaque d'extrémité (2).

2. L' implant rachidien préservant le mouvement pour le remplacement total du disque selon la revendication 1, comprenant :
la cavité interne traversant centralement et axialement le noyau externe (4) ;
et le noyau interne (3) étant positionné de manière concentrique à l'intérieur de la cavité interne.

3. L' implant rachidien préservant le mouvement pour le remplacement total du disque selon la revendication 1, dans lequel la première plaque d'extrémité (1), la seconde plaque d'extrémité (2), le noyau interne (3) et le noyau externe (4) ont chacun des géométries périphériques rectilignes.

4. L' implant rachidien préservant le mouvement pour le remplacement total du disque selon la revendication 1, comprenant :
le noyau interne (3) et le noyau externe (4) comprenant chacun un élément radial axe et un axe central ;
l'axe radial étant positionné décalé par rapport à l'axe central pour chacun du noyau interne (3) et du noyau externe (4) ; et la première convexité de noyau et
la seconde convexité de noyau du noyau interne (3) étant positionnées de manière concentrique autour de l'axe radial du noyau interne (3).

5. L' implant rachidien préservant le mouvement pour le remplacement total du disque selon la revendication 1, dans lequel le noyau externe (4) est relié entre la première plaque d'extrémité (1) et la seconde plaque d'extrémité (2) par l'intermédiaire d'un adhésif.

6. L' implant rachidien préservant le mouvement pour le remplacement total du disque selon la revendication 1, dans lequel le corps de plaque (20) de la première plaque d'extrémité (1) et le corps de plaque (20) de la seconde plaque d'extrémité (2) sont orientés selon un angle d'inclinaison spécifié l'un par rapport à l'autre, l'angle d'inclinaison spécifié définissant un écart du corps de plaque (20) de la première plaque d'extrémité (1) et du corps de plaque (20) de la seconde plaque d'extrémité (2) par rapport à une orientation parallèle l'un à l'autre; et dans lequel l'angle d'inclinaison spécifié est compris dans une plage de 0 à 15 degrés.

7. L' implant rachidien préservant le mouvement pour le remplacement total du disque selon la revendication 1, dans lequel le noyau interne (3) est constitué d'un matériau polymère.

8. L' implant rachidien préservant le mouvement pour le remplacement total du disque selon la revendication 1, dans lequel la première plaque d'extrémité (1) et la seconde plaque d'extrémité (2) sont constituées d'un matériau polyéther éther cétone (PEEK).

9. L' implant rachidien préservant le mouvement pour le remplacement total du disque selon la revendication 1, dans lequel le noyau interne (3) est constitué d'un caoutchouc de silicone liquide de dureté variable.

10. L' implant rachidien préservant le mouvement pour le remplacement total du disque selon la revendication 1, dans lequel le noyau externe (4) est constitué d'un caoutchouc de silicone liquide de dureté variable.

11. L' implant rachidien préservant le mouvement pour le remplacement total du disque selon la revendication 1, dans lequel le noyau externe (4) est constitué d'un matériau polymère.

12. L' implant rachidien préservant le mouvement pour le remplacement total du disque selon la revendication 1, comprenant :
la première plaque d'extrémité (1) et la seconde plaque d'extrémité (2) comprenant chacune en outre une bride de fixation (25) et au moins une ouverture de fixation (26) ;
la bride de fixation (25) étant reliée perpendiculairement et de façon périmétrique au corps de plaque et s'étendant à l'opposé du côté interne, au-delà du côté externe pour chacune de la première plaque d'extrémité (1) et de la seconde plaque d'extrémité (2), dans lequel la bride de fixation (25) est reliée le long d'un segment d'arc de bride du périmètre du corps de plaque ;
et la ou les ouvertures de fixation (26) traversant la bride de fixation (25) pour chacune de la première plaque d'extrémité (1) et de la seconde plaque d'extrémité (2).

13. L' implant rachidien préservant le mouvement pour le remplacement total du disque selon la revendication 12, comprenant :
la bride de fixation (25) comprenant une rainure intérieure (27) ;
et la rainure interne (27) traversant radialement la bride de fixation (25) le long du segment d'arc de bride adjacent au côté externe et adjacent à un périmètre du corps de plaque pour chacune de la première plaque d'extrémité (1) et de la seconde plaque d'extrémité (2).

14. L' implant rachidien préservant le mouvement pour le remplacement total du disque selon la revendication 1, comprenant :
le noyau externe (4) comprenant une première pluralité d'éléments à emboîtement de noyau et une seconde pluralité d'éléments à emboîtement de noyau parmi la pluralité d'éléments à emboîtement, la première pluralité d'éléments à emboîtement de noyau et la seconde pluralité d'éléments à emboîtement de noyau étant positionnées l'une en face de l'autre le long d'une épaisseur du noyau externe (4) ;
la première plaque d'extrémité (1) et la seconde plaque d'extrémité (2) comprenant chacune en outre une pluralité d'éléments à emboîtement de plaque parmi la pluralité d'éléments à emboîtement ;
la pluralité d'éléments à emboîtement de plaque étant positionnés de manière concentrique autour de la concavité sur le côté interne du corps de plaque pour chacune de la première plaque d'extrémité (1) et de la seconde plaque d'extrémité (2) ;
la première pluralité d'éléments à emboîtement de noyau du noyau externe (4) étant en prise avec la pluralité d'éléments à emboîtement de plaque de la première plaque d'extrémité (1) ;
et la seconde pluralité d'éléments à emboîtement de noyau du noyau externe (4) étant en prise avec la pluralité d'éléments à emboîtement de plaque de la seconde plaque d'extrémité (2).

15. L' implant rachidien préservant le mouvement pour le remplacement total du disque selon la revendication 14, comprenant :
une pluralité de canaux de réception d'éléments à emboîtement (7) ;
chacun de la pluralité d'éléments à emboîtement recevant des canaux (7) étant positionné de manière concentrique et adjacente à l'un de la pluralité d'éléments à emboîtement ;
et chacun de la pluralité d'éléments à emboîtement étant positionné dans l'un de la pluralité de canaux de réception d'éléments à emboîtement (7), la pluralité d'éléments à emboîtement et la pluralité de canaux de réception d'éléments à emboîtement (7) étant configurés pour résister à l'extrusion du noyau interne (3) et le noyau externe (4) lorsque le noyau interne (3) et le noyau externe (4) sont soumis à des forces externes.

16. L' implant rachidien préservant le mouvement pour le remplacement total du disque selon la revendication 6, comprenant :
le corps de plaque de la première plaque d'extrémité (1), le corps de plaque de la seconde plaque d'extrémité (2), le noyau interne (3) et le noyau externe (4) s'étendant chacun longitudinalement entre une extrémité proximale et une extrémité distale, dans lequel l'extrémité proximale et l'extrémité distale sont positionnées diamétralement opposées l'une à l'autre pour chacun du corps de plaque, du noyau interne (3) et du noyau externe (4),
les extrémités proximales étant alignées radialement l'une avec l'autre et les extrémités distales sont alignés radialement l'un avec l'autre pour chacun des corps de plaque de la première plaque d'extrémité (1) et de la seconde plaque d'extrémité (2), du noyau interne (3) et du noyau externe (4),
et dans lequel l'angle d'inclinaison spécifié est défini dans un plan coïncidant avec les extrémités proximales et les extrémités distales.

17. L' implant rachidien préservant le mouvement pour le remplacement total du disque selon la revendication 16, comprenant :
la première plaque d'extrémité (1) et la seconde plaque d'extrémité (2) comprenant chacune en outre une fixation de bride ; et la fixation de bride étant positionnée au niveau de l'extrémité proximale de chacune de la première plaque d'extrémité (1) et de la seconde plaque d'extrémité (2).

18. L' implant rachidien préservant le mouvement pour le remplacement total du disque selon la revendication 16, comprenant :
une épaisseur proximale du noyau interne (3) au niveau de l'extrémité proximale du noyau interne (3) étant supérieure à une épaisseur distale du noyau interne (3) au niveau de l'extrémité distale du noyau interne (3) ; et
une épaisseur proximale du noyau externe (4) au niveau de l'extrémité proximale du noyau externe (4) étant supérieure à une épaisseur distale du noyau externe (4) au niveau de l'extrémité distale du noyau externe (4), dans lequel l'angle d'inclinaison spécifié étant déterminé par la différence entre les épaisseurs proximales et les épaisseurs distales.
